# EUROPEAN PATENT SPECIFICATION

(11) **EP 3 816 277 B1**
(45) Date of publication and mention of the grant of the patent: **19.02.2025**
(21) Application number: 19825305.6
(22) Date of filing: 28.06.2019
(51) Int. Cl.: A01K 67/0276, A01K 67/0275, C07K 14/47, C12N 5/071, C12N 5/09, C12N 9/12, G01N 33/50

(54) **MUTANT MOUSE-DERIVED PANCREATIC ORGANOID AND METHOD FOR EVALUATING STANDARDIZED DRUG FOR EFFICACY**
PANKREATISCHES ORGANOID AUS MUTIERTER MAUS UND VERFAHREN ZUR EVALUIERUNG EINES STANDARDISIERTEN WIRKSTOFFS AUF SEINE WIRKSAMKEIT
ORGANOÏDE PANCRÉATIQUE DÉRIVÉ DE SOURIS MUTANTE ET PROCÉDÉ D'ÉVALUATION D'EFFICACITÉ D'UN MÉDICAMENT STANDARDISÉ

(30) Priority: 29.06.2018 KR 20180075560; 27.06.2019 KR 20190076841
(43) Date of publication of application: 05.05.2021
(73) Proprietor: Seoul National University R & DB Foundation, Seoul 08826 (KR)
(72) Inventor: LEE, Hyun Sook, Seoul 06552 (KR); JOO, So Young, Seoul 06585 (KR); PAIK, Sang Jin, Seoul 06520 (KR)
(74) Representative: Schnappauf, Georg
(86) International application number: PCT/KR2019/007892
(87) International publication number: WO 2020/005011

(56) References cited:
- WO-A1-2016/015158
- WO-A1-2018/230970
- WO-A2-97/35967
- KR-A- 20180 136 410
- ARGILLA DAVID ET AL: "Absence of telomerase and shortened telomeres have minimal effects on skin and pancreatic carcinogenesis elicited by viral oncogenes", CANCER CELL, vol. 6, no. 4, October 2004 (2004-10-01), US, pages 373 - 385, XP055890092, ISSN: 1535-6108, Retrieved from the Internet <URL:https://www.sciencedirect.com/science/article/pii/S1535610804002727/pdfft?md5=8ca794bbc101d7c531485d146453b8b1&pid=1-s2.0-S1535610804002727-main.pdf> DOI: 10.1016/j.ccr.2004.08.032
- SYLVIA F. BOJ ET AL: "Organoid Models of Human and Mouse Ductal Pancreatic Cancer", CELL, vol. 160, no. 1-2, 2015, Amsterdam NL, pages 324 - 338, XP055484794, ISSN: 0092-8674, DOI: 10.1016/j.cell.2014.12.021
- BAKER LINDSEY A. ET AL: "Modeling Pancreatic Cancer with Organoids", TRENDS IN CANCER, vol. 2, no. 4, April 2016 (2016-04-01), pages 176 - 190, XP055890079, ISSN: 2405-8033, Retrieved from the Internet <URL:https://www.ncbi.nlm.nih.gov/pmc/articles/PMC4847151/pdf/nihms774675.pdf> DOI: 10.1016/j.trecan.2016.03.004
- MATTHEW ROWLEY ET AL: "Inactivation of Brca2 Promotes Trp53-Associated but Inhibits KrasG12D-Dependent Pancreatic Cancer Development in Mice", GASTROENTEROLOGY, ELSEVIER INC, US, vol. 140, no. 4, 20 December 2010 (2010-12-20), pages 1303 - 1313.e3, XP028156897, ISSN: 0016-5085, [retrieved on 20110101], DOI: 10.1053/J.GASTRO.2010.12.039
- M. L. NICKERSON ET AL: "Concurrent Alterations in TERT, KDM6A, and the BRCA Pathway in Bladder Cancer", CLINICAL CANCER RESEARCH, vol. 20, no. 18, 15 September 2014 (2014-09-15), US, pages 4935 - 4948, XP055584078, ISSN: 1078-0432, DOI: 10.1158/1078-0432.CCR-14-0330
- KWON MI-SUN ET AL: "Brca2 abrogation engages with the alternative lengthening of telomeres via break-induced replication", THE FEBS JOURNAL, vol. 286, no. 10, 27 February 2019 (2019-02-27), GB, pages 1841 - 1858, XP055890037, ISSN: 1742-464X, Retrieved from the Internet <URL:https://onlinelibrary.wiley.com/doi/full-xml/10.1111/febs.14796> DOI: 10.1111/febs.14796
- YANG, TING-LIN B.: "Mutual reinforcement between telomere capping and canonical Wnt signalling in the intestinal stem cell niche", NATURE COMMUNICATIONS, vol. 8, no. 1, 17 March 2017 (2017-03-17), pages 14766, XP055667032
- MARTINEZ-USEROS, JAVIER: "The role of BRCA2 mutation status as diagnostic, predictive, and prognosis biomarker for pancreatic cancer", BIOMED RESEARCH INTERNATIONAL, vol. 2016, 18 December 2016 (2016-12-18), pages 1869304, XP055667035
- HOU, SHURONG ET AL: "Advanced development of primary pancreatic organoid tumor models for high-throughput phenotypic drug screening", SLAS DISCOVERY: ADVANCING LIFE SCIENCES R&D, vol. 23, no. 6, 19 April 2018 (2018-04-19), pages 575 - 584, XP055667040
- BRUEDIGAM, CLAUDIA: "Telomerase inhibition effectively targets mouse and human AML stem cells and delays relapse following chemotherapy", CELL STEM CELL, vol. 15, no. 6, 4 December 2014 (2014-12-04), pages 775 - 790, XP055667043

## Description

### Technical Field

The present disclosure relates to a method for producing three-dimensional pancreatic organoids derived from the pancreas of a genetically mutated mouse.

### Background Art

Most of conventional anticancer therapeutic drugs developed through new drug development studies have not been actively applied to actual cancer patients due to their side effects or low efficacy in clinical practice. In addition, even patients with the same cancer have different responses to the same anticancer therapeutic drug in many cases, and thus studies on such responses are urgently needed. Completion of the human genome project has accumulated sequencing-related reference information for human genetic information (Lander E. S., Linton L. M., Birren B., Nusbaum C., Zody M. C., Baldwin J., et al. (2001). Initial sequencing and analysis of the human genome. Nature 409, 860-921. 10.1038/35057062). As a result, it is expected that whole-genome sequencing (WGS) for humans has been conducted and will gradually reach an accessible level and be more actively used for cancer research in the future. However, information on cancer cell tissue derived through conventional two-dimensional cell culture methods does not accurately reflect the *in vivo* situation, and thus the demand for new cell culture methods is growing. A cell culture method that can meet this demand is a three-dimensional organoid culture method on which the present disclosure is based. The three-dimensional organoid refers to stem cell-derived cells growing in a three-dimensional structure to form cells that mimic a specific organ and self-organize (Clevers, H. 2016. Modeling development and disease with organoids. Cell. 165:1586-1597. http://dx.doi.org/10.1016/j.cell.2016.05.082). This three-dimensional organoid is a system that contains complete genetic information and is capable of actually modeling cancer more closely to *in vivo* conditions.

The most important thing in cancer treatment research will be *in vitro* and *in vivo* animal models mimicking cancer. Through constructed models, researchers may conduct research related to cancer treatment to understand cancer treatment. The most basic method used for cancer modeling is cell culture. A method that has been most actively used to date is a method of constructing an animal model so that observation and experiments may be performed *in vitro* by continuously culturing a cancer cell line, derived from mouse or human tissue, in a monolayer state. This two-dimensional culture method has a disadvantage in that it cannot reflect the *in vivo* situation in which cells actually exist in three dimensions (Lee et al., 2008; Vunjak-Novakovic and Freed, 1998). On the other hand, cells cultured through the three-dimensional organoid culture method have genetic information that is almost similar to cancer cell tissues derived from actual patients (Dong Gao et al., 2014. Organoid Cultures Derived from Patients with Advanced Prostate Cancer, Cell, 176-187, https://doi.org/10.1016/j.cell.2014.08.016). Therefore, applied research using these three-dimensional organoids will be a foothold that provides personalized medicine to actual patients.

International Patent Application Publication No. WO 97/35967 A2 discloses non-human transgenic organisms in which telomerase activity is altered.

Argilla et al., 2004, Cancer Cell 6:373-385 disclose that the absence of telomerase and shortened telomeres have minimal effect on skin and pancreatic carcinogenesis elicited by viral oncogenes.

Boj et al., 2015, Cell 160:324-338 disclose organoid models of human and mouse ductal pancreatic cancer.

Baker et al., 2016, Trends Cancer 2:176-190 disclose modelling pancreatic cancer with organoids.

Rowley et al., 2011, Gastroenterology 140:1303-1313 disclose that inactivation of Brca2 promotes Trp53-asssociated but inhibits KrasG12D-dependent pancreatic cancer development in mice.

Nickerson et al., 2014, Clin Cancer Res 20:4935-4948 discuss concurrent alterations in *TERT, KDM6A,* and the BRCA pathway in bladder cancer.

### DISCLOSURE

### Technical Problem

The present invention is directed to a method for producing pancreatic organoids capable of continuous culture, the method comprising the steps of: (a) isolating pancreatic tissue from a mouse, wherein the mouse is genetically modified by knockout of the telomerase RNA component (TERC) and by the deletion of exon 11 of the BRCA2 gene; (b) mixing cells isolated from the tissue with Matrigel, and culturing the mixed cells to form pre-organoids; (c) dissociating the pre-organoids with a mechanical stress of 50 to 500 Pa, and (d) re-culturing the dissociated organoids by repeated passaging.

In an embodiment of the present invention, the organoids have a diameter of 100 to 200 µm.

The present invention is defined by the subject-matter set out in the appended claims. However, the following disclosure provides teachings which go beyond the disclosure of the invention as such, which is defined exclusively by the appended claims. The teachings are provided to place the actual invention in a broader technical context and to illustrate possible related technical developments. Such additional technical information which does not fall within the scope of the appended claims is not part of the invention. In particular, the terms "embodiment", "invention", and "aspect" are not to be construed as necessarily referring to the claimed invention, unless the subject-matter in question falls within the scope of the claims. For example, embodiments directed to an animal model or cancer treatment are not encompassed by the wording of the appended claims.

The present disclosure has been made in order to solve the above-described problems occurring in the prior art, and relates to three-dimensional pancreatic organoids derived from a genetically modified mouse and to a standardized method for evaluating drug effect.

Applied research using these three-dimensional organoids will be a foothold that provides personalized medicine to actual patients by reflecting the *in vivo* situation in which cells actually exist in three dimensions.

However, technical objects to be achieved by the present disclosure are not limited to the above-mentioned object, and other objects which are not mentioned herein will be clearly understood by those skilled in the art from the following description.

One example provides three-dimensional pancreatic organoids derived from the pancreas of a genetically mutated mouse.

Another example provides a method for producing three-dimensional pancreatic organoids, the method including a step of three dimensionally culturing pancreatic cells of a genetically mutated mouse.

Still another example provides a composition for verifying drug effect, the composition containing the three-dimensional pancreatic organoids derived from the pancreas of a genetically mutated mouse.

Yet another example provides a method for verifying drug effect or a method of providing information for verifying drug responsiveness, the method including a step of treating the three-dimensional pancreatic organoids, derived from the pancreas of a genetically mutated mouse, with the drug.

Still yet another example provides a composition for drug screening, the composition containing the three-dimensional pancreatic organoids derived from the pancreas of a genetically mutated mouse.

A further example provides a method for drug screening, the method including a step of treating the three-dimensional pancreatic organoids, derived from the pancreas of a genetically mutated mouse, with a candidate substance.

Further another example provides the use (or a method of use) of organoids as a cancer model, the use including a step of karyotyping the three-dimensional pancreatic organoids derived from the pancreas of the genetically modified mouse and comparing the karyotype pattern of the organoids depending on the genotype thereof with a wild type.

Still further another example provides a method for identifying growth of organoids depending on the genotype thereof, the method including a step of observing the division rate and pattern of the three-dimensional pancreatic organoids derived from the pancreas of the genetically modified mouse.

Yet further another example provides a method for producing organoids for use in measurement of drug responsiveness or in drug screening. The method for producing organoids may include the following steps:
(1) culturing stem cells with a biomatrix to produce organoids;
(2) collecting organoids having an average diameter of 1,000 µm or less among the produced organoids; and
(3) allowing the collected organoids to grow.

Still yet further another example provides a composition or kit for measurement (prediction or verification) of drug responsiveness or for drug screening, the composition or kit containing organoids prepared by the method for producing organoids for use in measurement of drug responsiveness or in drug screening.

Another example provides a method for measurement (prediction or verification) of drug responsiveness or for drug screening, the method including a step of treating organoids, prepared by the method for producing organoids for use in measurement of drug responsiveness or in drug screening, with the drug. The method may further include steps (1) to (3) of the above-described method for producing organoids, before treating the organoids with the drug.

The composition or kit or method for use in measurement (prediction or verification) of drug responsiveness or for drug screening, which is provided in the present specification, may be a standardized technology that is universally applicable to all types of organoids regardless of the origin, form, etc. of the organoids.

### Technical Solution

Hereinafter, various embodiments described herein will be described with reference to figures. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present disclosure pertains.

The present disclosure aims at modeling cancer closely to the *in vivo* state using a gene analysis method such as whole-genome sequencing (WGS) and organoids obtained from the normal tissue and cancer tissue of a genetically modified mouse, and usefully applying the organoids for drug efficacy verification depending on genetic modification. The present disclosure is intended to provide a new method for verifying an anticancer therapeutic drug, through treatment with a genotype-specific drug, using whole-genome sequencing (WGS) and organoids derived from genetically engineered mice or human patients. Among pancreatic organoids derived from genetically modified mice provided in the present specification, organoids having respective genotypes may be cultured for a long period of time, whether or not other genetic modifications are involved may be examined through whole-genome sequencing (WGS), and accumulation of genetic modifications in a carcinogenesis process may be compared.

An object of the present disclosure is to provide three-dimensional organoids derived from the pancreas of a genetically modified mouse, the organoids being capable of simulating a biological tissue, in particular, a cancer tissue, which exists in three dimensions *in vivo,* in a manner similar to an *in vivo* situation.

The term "organoid" means a three-dimensional cell aggregate formed through self-renewal and self-organization from adult stem cells (ASCs), embryonic stem cells (ESCs), induced pluripotent stem cells (iPSCs), or the like. Cells may be aggregated again and recombined by a three-dimensional culture method, so that they may be made similar to those *in vivo,* whereby they can overcome limitations of 2-D cell lines cultured by a 2-D culture method and similarly reproduce physiological activity functions of a living body. Thus, the organoids are applicable to disease modeling, drug screening, and the like.

As used herein, the term "pancreatic organoid" refers to a three-dimensional cell aggregate obtained by culturing pancreatic cells (e.g., pancreatic adult stem cells) using a three-dimensional culture method.

One example provides three-dimensional pancreatic organoids derived from the pancreas of a genetically mutated mouse.

In the present specification, the term "genetically mutated mouse" refers to a mouse having at least one mutation selected from the following mutations:
(1) BRCA2 gene mutation;
(2) knockout of telomerase RNA component (TERC);
(3) genetic mutation which induces mutation of the acetylation residue of BubR1 protein; and
(4) KRAS gene mutation.

In one embodiment, the genetically mutated mouse may contain:
(1) BRCA2 gene mutation;
(2) BRCA2 gene mutation and knockout of telomerase RNA component (TERC);
(3) genetic mutation which induces mutation of the acetylation residue of BubR1 protein; or
(4) KRAS gene mutation.

The BRCA2 (breast cancer 2) gene is a gene located on chromosome 5 (Chr 5: 150.52-150.57 Mb) of mice (*Mus musculus*) and may be selected from GebBank Accession No. NM_001081001.2 (NP_001074470.1-encoding gene), GenBank Accession No. NM_009765.3 (NP _033895.2-encoding gene), and the like. In the genetically mutated mouse, BRCA2 gene mutation may mean complete deletion of exon 11 of the BRCA2 gene. In one embodiment, the genetically mutated mouse may be, but is not limited to, a mouse in which specific conditional deletion occurs, for example, a mouse in which Cre recombinase-mediated deletion of exon 11 is induced in an allele (e.g., conditional deletion is induced by a tamoxifen-like compound such as 4-hydroxytamoxifen). To induce the Cre recombinase-mediated deletion of exon 11, the genetically mutated mouse may be a mouse into which the Cre recombinase gene has been introduced. For example, introduction of the Cre recombinase gene may be performed by crossing of a mouse in which exon 11 of the BRCA2 gene has been deleted with a mouse into which the Cre recombinase gene has been inserted, introduction of a conventional vector (e.g., an adenovirus vector) containing the Cre recombinase gene by a conventional method, or the like, but is not limited thereto. The Cre recombinase may be derived from Bacteriophage P1, and may be represented by UniProtKB P06956 (GenBank Accession No. YP_006472.1; coding gene (mRNA): CDS (436..1467) of NC_005856.1)), but is not limited thereto.

The telomerase RNA component (TERC) refers to non-coding RNA (ncRNA) present in eukaryotic cells, which is an RNA component of telomerase (ribonucleoprotein) having telomere elongation activity. The telomere is a specific form of genetic material that is repeatedly present at the end of each chromosome. The telomere has a function of preventing chromosomal damage or binding to other chromosomes. Whenever cells divide, the length of the telomere becomes shorter slightly. When the number of cell division cycles exceeds a certain number, the length of the telomere becomes very short, and the corresponding cell stops division and dies. This is a natural phenomenon called "apoptosis" in which old or damaged cells commit suicide by themselves in order not to cause problems, and thus telomeres are sometimes referred to as "life clock". On the other hand, germ cells and cancer cells excluding somatic cells are capable of infinite proliferation because the telomere does not shorten. In particular, cancer cells do not die and continue to proliferate, because telomerase is secreted and prevents the telomeres from shortening. Thus, when the telomerase is knocked out, the telomere extension activity is suppressed, so that carcinogenesis does not proceed, and cancer cells become normal. That is, when TERC is normal, carcinogenesis proceeds, and when TERC is knocked out, cancer cells become normal. For example, the WRN gene is a gene associated with accelerated aging and the level thereof increases in cancer. As another example, the BLM gene is a cancer-related gene that causes genomic instability when mutated, and causes Bloom syndrome characterized by cancer. There are research results indicating that, even if WRN and BLM genes are additionally knocked out or mutated in a state in which cancer cells have become normal through knockout of TERC, carcinogenesis does not proceed and the cancer cells are still maintained in a normal cell state (Telomere Shortening Exposes Functions for the Mouse Werner and Bloom Syndrome Genes, 2004).

In the present disclosure, knockout of the telomerase RNA component in the genetically modified mouse may refer to deletion of one or more RNAs or substitution with a different nucleotide than the original nucleotide in the full-length RNA sequence (397 nt) of the telomerase RNA component, and examples thereof include knockout (deletion) of the full-length RNA sequence, replacement of the TERC gene using a targeting vector, and the like.

Carcinogenesis in the mouse with deletion of both the TERC and the BRCA2 gene according to the present disclosure was induced in a manner different from that in a conventional mouse with deletion of the TERC.

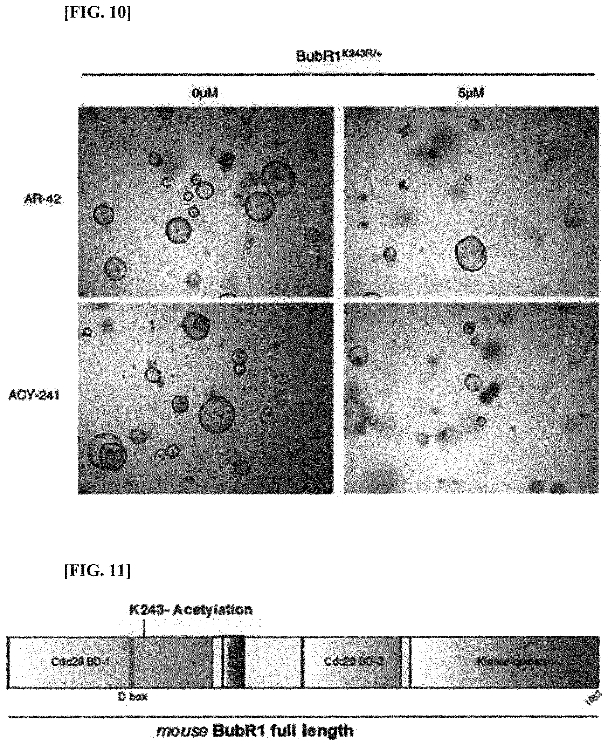

The BubR1 (mitotic checkpoint serine/threonine kinase; Bub1b) is a gene which encodes a kinase involved in spindle checkpoint function and chromosomal segregation. The BubR1 is located at the kinetochore, and acts to inhibit anaphase-promoting complex/cyclosome (APC/C) and delay entry into anaphase. Spindle checkpoint dysfunction is observed in various cancers. The mouse (*Mus musculus*) BubR1 gene may be GebBank Accession No. NM_009773.3 (NP_033903.2-encoding gene). The structure of mouse BubR1 protein is schematically shown in FIG. 11.

In the genetically mutated mouse, the BubR1 gene may be modified to encode a BubR1 protein having a substitution at the acetylation residue. The acetylation residue may be, for example, the 243^{rd} amino acid residue lysine (K) (K243) in the amino acid sequence of NP_033903.2, and the mutation may be mutation that inhibits acetylation at the acetylation residue (for example, K243). In one embodiment, the BubR1 gene mutation may be done such that the BubR1 gene encodes a modified BubR1 protein in which K243 of BubR1 protein has been modified into arginine (R).

The KRAS is a gene that functions as a switch in cell signaling. While the KRAS functions normally, it regulates cell proliferation, but when the KRAS is mutated, signaling is stopped and the cells continue to proliferate, usually leading to cancer. Thus, this KRAS gene is called proto-oncogene. KRAS^{G12D} mutation is frequently observed. The mouse (*Mus musculus*) KRAS gene may be GenBank Accession No. NM_021284.6 (NP_067259.4-encoding gene).

Organoids generated from the pancreas of the above-described genetically modified mouse have an excellent ability to simulate an *in vivo* environment compared to a wild-type mouse (a mouse that does not have the above-mentioned modification), and in particular, can more closely simulate an *in vivo* cancer environment. Thus, the organoids can be more advantageously applied to tests for the effects of various drugs (responsiveness to drugs) such as anticancer drugs and/or drug screening.

Another example provides a method for producing three-dimensional pancreatic organoids, the method including a step of three dimensionally culturing pancreatic cells of a genetically mutated mouse.

The genetically mutated mouse is as described above.

The step of three dimensionally culturing pancreatic cells may include steps of:
(i) dissociating a pancreatic tissue isolated from the genetically mutated mouse;
(ii) collecting cell pellets containing ductal cells from the dissociated pancreatic tissue; and
(iii) culturing the collected cell pellets with a biomatrix.

Step (i)) of dissociating the pancreatic tissue may be performed by treating the pancreatic tissue with a dissociation solution. The dissociation solution may contain Hank's Balanced Salt Solution (HBSS) (e.g., 1 ml to 10 ml, 1 ml to 5 ml, 3 ml to 10 ml, or 3 to 5 ml), collagenase (e.g., collagenase P) (e.g., 0.1 mg/ml to 5 mg/ml, 0.1 mg/ml to 3 mg/ml, 0.5 mg/ml to 5 mg/ml, 0.5 mg/ml to 2 mg/ml, or 0.8 mg/ml to 1.5 mg/ml), DNase (e.g., DNase 1) (e.g., 0.01 mg/ml to 1 mg/ml, 0.01 mg/ml to 0.5 mg/ml, 0.01 mg/ml to 0.2 mg/ml, 0.05 mg/ml to 1 mg/ml, 0.05 mg/ml to 0.5 mg/ml, 0.05 mg/ml to 0.2 mg/ml, or 0.08 mg/ml to 0.15 mg/ml), and the like. In one example, the dissociation solution may be used in an amount of 0.5 to 5 ml, 0.5 to 4 ml, 0.5 to 3 ml, 1 to 5 ml, 1 to 4 ml, or 1 to 3 ml relative to the pancreatic tissue (Vpan=about 1.08 mg/mm³). However, the amount of the dissociation solution is not limited thereto, and may be appropriately regulated and used depending on the amount of the pancreatic tissue. At this time, in order to facilitate dissociation of the tissue, physical dissociation (e.g., a step of performing fine cutting with scissors, a knife, or the like) and treatment with the dissociation solution may be performed at the same time or at different times in any order.

Step (ii) of collecting the cell pellets containing ductal cells may include steps of: (ii-1) isolating ductal cells from the dissociated pancreatic tissue; and (ii-2) centrifuging the isolated ductal cells to collect cell pellets. Step (ii-1) of isolating ductal cells from the dissociated pancreatic tissue may include a step of filtering the dissociated pancreatic tissue obtained in the step (i), and separating and collecting ductal cells among the cells that did not pass through the filter. The filtration may be performed using a conventional cell strainer having a pore size (e.g., 50 µm to 200 µm, 50 µm to 170 µm, 50 µm to 150 µm, 50 µm to 120 µm, 80 µm to 200 µm, 80 µm to 170 µm, 80 µm to 150 µm, 80 µm to 120 µm, or 90 µm to 110 µm) which does not pass ductal cells. Separation of the ductal cells may be performed by conventional cell identification means such as microscopic observation. Step (ii-2) of collecting the cell pellets may include a step of collecting pellets by centrifuging the ductal cells isolated in step (ii-1), for example, at 1000 rpm to 2000 rpm, 1000 rpm to 1800 rpm, 1000 rpm to 1600 rpm, 1200 rpm to 2000 rpm, 1200 rpm to 1800 rpm, 1200 rpm to 1600 rpm, 1400 rpm to 2000 rpm, 1400 rpm to 1800 rpm, or 1400 rpm to 1600 rpm, for 1 to 20 minutes, 1 to 15 minutes, 1 to 12 minutes, 5 to 20 minutes, 5 to 15 minutes, 5 to 12 minutes, 8 to 20 minutes, 8 to 15 minutes, or 8 to 12 minutes.

In step (iii) of culturing the collected cell pellets with a biomatrix, the biomatrix may include one or more selected from the group consisting of Matrigel, extracellular matrix (ECM), Basement Membrane Extract (BME), and hyaluronic acid. The Matrigel refers to a mixture of gelatin-like proteins secreted from Engelbreth-Holm-Swarm (EHS) mouse sarcoma cells. The extracellular matrix is a cluster of biopolymers containing molecules which are synthesized in cells and secreted and accumulated outside the cells, and may include one or more selected from the group consisting of fibrotic proteins such as collagen and elastin, complex proteins such as glycosaminoglycan, and cell adhesion proteins such as fibronectin and laminin.

The culturing in step (iii) may be performed under conventional cell culture conditions, for example, conditions of 35 to 36°C and 8 to 12% CO₂ for 1 to 10 days, 1 to 7 days, 3 to 10 days, or 3 to 7 days.

The method may include a step of collecting a portion of the organoid tissue obtained by culturing in step (iii) and applying mechanical stress thereto. When an object receives an external force, an internal resistance force that resists this force is generated. In the present disclosure, the mechanical stress refers to the size per unit area at each point in the object. The step of applying mechanical stress refers to physically dissociating the organoid tissue, selected in the first culture step, through two steps, and then culturing the organoids again.

The above-described three-dimensional pancreatic organoids derived from the pancreas of the genetically mutated mouse may be produced by the method for producing three-dimensional pancreatic organoids.

Still another example provides a composition for verifying drug effect, the composition containing the three-dimensional pancreatic organoids derived from the pancreas of a genetically mutated mouse. Yet another example provides a method for verifying drug effect or a method of providing information for verifying drug effect, the method including a step of treating the three-dimensional pancreatic organoids, derived from the pancreas of a genetically mutated mouse, with a drug. The drug may be a drug for treating a pancreatic disease, for example, a drug for treating a pancreatic disease associated with the above-described BRCA2 mutation, telomerase RNA component (TERC) mutation, BubR1 mutation and/or KRAS mutation, for example, an anti-cancer drug against pancreatic cancer. In one embodiment, the drug may be, but is not limited to, one or more selected from the group consisting of histone deacetylase inhibitors (HDACi; e.g., Trichostatin A (TSA), suberoylanilide hydroxamic acid (SAHA), LMK-235, FK-228 (Romidepsin), etc.), PARP-1 (poly[ADP-ribose] polymerase 1) inhibitors (e.g., Olaparib, Veliparib (ABT-888), Iniparib (BSI-201), etc.), and polo-like kinase 1 (plk 1) inhibitors (e.g., BI2536, Volasertib (BI 6727), Rigosertib (ON-01910), etc.). The drug effect means a biological action to be achieved by the drug, and may refer to an effect such as mitigation, alleviation or treatment of the pancreatic disease (for example, pancreatic cancer). In a case where the pancreatic disease is pancreatic cancer, the drug effect may refer to mitigation, elimination, or inhibition of progression or metastasis of pancreatic cancer.

In a case where the size and/or number of the three-dimensional pancreatic organoids decrease(s) after the organoids are treated with a drug, it may be determined (or judged) that the drug has an effect against a pancreatic disease (e.g., pancreatic cancer). Accordingly, the method for verifying drug effect or the method of providing information for verification may include, after the step of treating the organoids with the drug, a step of measuring the size and/or number of the three-dimensional pancreatic organoids treated with the drug, and comparing the measurement result with that of three-dimensional pancreatic organoids not treated with the drug. To this end, the comparing step may further include a step of measuring the size and/or number of the three-dimensional pancreatic organoids not treated with the drug, and this step may be performed simultaneously with or at a different time than the step of measuring the size and/or number of the three-dimensional pancreatic organoids treated with the drug. The three-dimensional pancreatic organoids not treated with the drug may be the three-dimensional pancreatic organoids before treatment with the drug, or some three-dimensional pancreatic organoids remaining after treating some of the three-dimensional pancreatic organoids with the drug. In addition, the method for verifying drug effect or the method of providing information for verification may further include, after the comparing step, a step of determining (or judging) that the drug has an effect against a pancreatic disease (e.g., pancreatic cancer) when the size and/or number of the three-dimensional pancreatic organoids treated with the drug decrease(s) compared to that of the three-dimensional pancreatic organoids not treated with the drug.

Still yet another example provides a composition for drug screening, the composition containing the three-dimensional pancreatic organoids derived from the pancreas of a genetically mutated mouse.

A further example provides a method for drug screening, the method including a step of treating the three-dimensional pancreatic organoids, derived from the pancreas of a genetically mutated mouse, with a candidate substance. The candidate substance may be selected among all bioactive substances, and may be, for example, one or more selected from the group consisting of bioactive small molecular chemicals, peptides, proteins (e.g., antibodies, other protein drugs, etc.), nucleic acid molecules, and extracts (e.g., animal or plant extracts). The drug screening method may be a method for screening a drug having a therapeutic effect against a pancreatic disease. The pancreatic disease may be a pancreatic disease associated with the above-described BRCA2 mutation, telomerase RNA component (TERC) mutation, BubR1 mutation, and/or KRAS mutation, for example, pancreatic cancer.

In a case where the size and/or number of the three-dimensional pancreatic organoids decrease(s) after treatment of the organoids with a candidate substance, it may be determined (or judged) that the candidate substance is a drug having an effect against a pancreatic disease (e.g., pancreatic cancer). Thus, the screening method may include, after the step of treating the organoid with the candidate substance, a step of measuring the size and/or number of the three-dimensional pancreatic organoids treated with the candidate substance, and comparing the measurement result with that of three-dimensional pancreatic organoids not treated with the candidate substance. To this end, the comparing step may further include a step of measuring the size and/or number of the three-dimensional pancreatic organoids not treated with the candidate substance, and this step may be carried out, simultaneously with or at a different time than the step of measuring the size and/or number of the three-dimensional pancreatic organoids treated with the candidate substance. The three-dimensional pancreatic organoids not treated with the candidate substance may be the three-dimensional pancreatic organoids before treatment with the candidate substance, or some three-dimensional pancreatic organoids remaining after treating some of the three-dimensional pancreatic organoids with the candidate substance. In addition, the screening method may further include, after the comparing step, a step of determining (or judging) that the drug is a candidate drug having an effect against a pancreatic disease (e.g., pancreatic cancer) when the size and/or number of the three-dimensional pancreatic organoids treated with the candidate substance decrease(s) compared to the size and/or number of the three-dimensional pancreatic organoids not treated with the candidate substance.

Further another example provides the use (or a method of use) of organoids as a cancer model, the use including a step of karyotyping the three-dimensional pancreatic organoids derived from the pancreas of the genetically modified mouse and comparing the karyotype pattern of the organoids depending on the genotype thereof with a wild type.

Still further another example provides a method for identifying growth of organoids depending on the genotype thereof, the method including a step of observing the division rate and pattern of the three-dimensional pancreatic organoid derived from the pancreas of the genetically modified mouse.

Yet further another example provides a method for producing organoids for use in measurement of responsiveness to a drug or in drug screening. The method for producing organoids may include steps of:
(1) culturing stem cells with a biomatrix to produce organoids;
(2) collecting organoids having an average diameter of 1,000 µm or less, 900 µm or less, 800 µm or less, 700 µm or less, 600 µm or less, 500 µm or less, 400 µm or less, 300 µm or less, or 200 µm or less, among the produced organoids; and
(3) allowing the collected organoids to grow.

In step (1), the stem cells may be selected from among all types of stem cells capable of forming three-dimensional organoids through self-renewal and self-organization, and may be, for example, at least one type of stem cells selected from the group consisting of adult stem cells (ASCs), embryonic stem cells (ESCs), induced pluripotent stem cells (iPSCs), and progenitor cells.

The embryonic stem cells are stem cells that can be derived from fertilized eggs, which are capable of differentiating into cells of any tissue. The induced pluripotent stem cells (iPS cells) are also called dedifferentiated stem cells, and refer to cells with induced pluripotency, like embryonic stem cells, which are obtained by injecting a cell differentiation-related gene into differentiated somatic cells to thereby return the somatic cells to the cell stage before differentiation. The progenitor cells have the ability to differentiate into a specific type of cells, similar to stem cells, but they are more specific and targeted than stem cells, and the number of divisions thereof is limited, unlike stem cells. The progenitor cells may be, but are not limited to, mesenchymal-derived progenitor cells. In the present specification, the progenitor cells are included in the scope of stem cells, and unless otherwise specified, the term "stem cells" is interpreted as including progenitor cells as well.

The adult stem cells are stem cells extracted from umbilical cords, umbilical cord blood, or adult bone marrow, blood, nerves, organs, epithelium or muscles, and refer to primitive cells just before differentiation into cells of a specific organ. The adult stem cells may be one or more types selected from the group consisting of hematopoietic stem cells, mesenchymal stem cells, and neural stem cells. The mesenchymal stem cells (MSCs), also called mesenchymal stromal cells (MSCs), refer to multipotent stromal cells capable of differentiating into various types of cells such as osteoblasts, chondrocytes, myocytes, adipocytes, epithelial cells, or nerve cells. The mesenchymal stem cells may be selected from among pluripotent cells derived from non-marrow tissues such as epithelial tissue, placenta, umbilical cord, umbilical cord blood, adipose tissue, adult muscle, corneal stroma, or dental pulp of milk teeth.

The stem cells may be derived from mammals such as primates (e.g., humans, monkeys, etc.), and rodents (e.g., mice, rats, etc.), and may be isolated from normal or diseased regions of *in vivo* organs or tissues.

The stem cells in step (1) may be obtained by dissociating tissues (e.g., tissues of specific organs such as pancreas, stomach, intestines, etc.) isolated from mammals. Thus, the method may further include a step of dissociating the tissue, before step (1). The dissociation step may be performed by treating the tissue with a dissociation solution. The dissociation solution may contain HBSS (Hank's Balanced Salt Solution) (e.g., 1 ml to 10 ml, 1 ml to 5 ml, 3 ml to 10 ml, or 3 ml to 5 ml), collagenase (e.g., collagenase P) (e.g., 0.1 mg/ml to 5 mg/ml, 0.1 mg/ml to 3 mg/ml, 0.5 mg/ml to 5 mg/ml, 0.5 mg/ml to 2 mg/ml, or 0.8 mg/ml to 1.5 mg/ml), DNase (e.g., DNase 1) (e.g., 0.01 mg/ml to 1 mg/ml, 0.01 mg/ml to 0.5 mg/ml, 0.01 mg/ml to 0.2 mg/ml, 0.05 mg/ml to 1 mg/ml, 0.05 mg/ml to 0.5 mg/ml, 0.05 mg/ml to 0.2 mg/ml, or 0.08 mg/ml to 0.15mg/ml), etc. In one example, the dissociation solution may be used in an amount of 0.5 to 5 ml, 0.5 to 4 ml, 0.5 to 3 ml, 1 to 5 ml, 1 to 4 ml, or 1 to 3 ml relative to tissue (Vpan= about 1.08 mg/mm³). However, the amount of the dissociation solution is not limited thereto, and may be appropriately regulated and used depending on the amount of the pancreatic tissue. At this time, in order to facilitate dissociation of the tissue, physical dissociation (e.g., a step of performing fine cutting with scissors, a knife, or the like) and treatment with the dissociation solution may be performed at the same time or sequentially in any order.

The stem cells may be isolated from the dissociated tissue obtained in the step of dissociating the tissue. The stem cells may be contained in pellets obtained by passing the dissociated tissue through a conventional cell strainer having a predetermined pore size (e.g., 50 µm to 200 µm, 50 µm to 170 µm, 50 µm to 150 µm, 50 µm to 120 µm, 80 µm to 200 µm, 80 µm to 170 µm, 80 µm to 150 µm, 80 µm to 120 µm, or 90 µm to 110 µm) and centrifuging the filtration retentate that does not pass through the cell strainer. The centrifugation may be performed at 1000 rpm to 2000 rpm, 1000 rpm to 1800 rpm, 1000 rpm to 1600 rpm, 1200 rpm to 2000 rpm, 1200 rpm to 1800 rpm, 1200 rpm to 1600 rpm, 1400 rpm to 2000 rpm, 1400 rpm to 1800 rpm, or 1400 rpm to 1600 rpm for 1 to 20 minutes, 1 to 15 minutes, 1 to 12 minutes, 5 to 20 minutes, 5 to 15 minutes, 5 to 12 minutes, 8 to 20 minutes, 8 to 15 minutes, or 8 to 12 minutes.

Therefore, the method may further include a step of collecting the pellets obtained by filtering and centrifuging the dissociated tissue as described above, before step (1) and after the step of dissociating the tissue.

The stem cells in step (1) may be in the form of cell pellets obtained by centrifuging the filtration retentate (retentate remaining without being filtered) obtained by dissociating a tissue isolated from a mammal and filtering the dissociated tissue.

In one embodiment, the stem cells may be isolated from the above-described genetically mutated mouse or the organ (e.g., pancreas) of the genetically mutated mouse, but are not limited thereto and may be selected from all organoids regardless of the origin thereof.

In step (1), the biomatrix refers to a material that creates an environment similar to an *in vivo* environment to enable three-dimensional culture of stem cells in the environment similar to the *in vivo* environment. The biomatrix may include one or more selected from the group consisting of Matrigel, extracellular matrix (ECM), Basement Membrane Extract (BME), and hyaluronic acid. The Matrigel refers to a mixture of gelatin-like proteins secreted from Engelbreth-Holm-Swarm (EHS) mouse sarcoma cells. The extracellular matrix is a cluster of biopolymers containing molecules which are synthesized in cells and secreted and accumulated outside the cells, and may include one or more selected from the group consisting of fibrotic proteins such as collagen and elastin, complex proteins such as glycosaminoglycan, and cell adhesion proteins such as fibronectin and laminin.

The culturing in step (1) may be performed under conventional cell culture conditions, for example, conditions of 35 to 38°C and 3 to 10% CO₂. The culturing may be performed until the average diameter of the organoids reaches 1000 µm or less, 900 µm or less, 800 µm or less, 700 µm or less, 600 µm or less, 500 µm or less, 400 µm or less, 300 µm or less, or 200 µm or less. For example, the culturing may be performed for 1 to 14 days, 1 to 10 days, 1 to 7 days, 3 to 14 days, 3 to 10 days or 3 to 7 days, followed by one or more passages, with 1 to 14 days, 1 to 10 days, 1 to 7 days, 3 to 14 days, 3 to 10 days, or 3 to 7 days per passage, so that the culturing can be continuously performed for up to 12 months, 9 months, or 6 months. A medium that is used for the culture may contain, but is not limited to, one or more selected from the group consisting of DMEM/F-12 (Dulbecco's Modified Eagle Medium/Ham's F-12), penicillin/streptomycin, Hank's Balanced Salt Solution (HEPES), GlutaMAX, N-acetylcysteine, conditioned media (e.g., Rspo 1-conditioned medium), nicotinamide, gastrin (e.g., gastrin I), growth factors (e.g., EGF, FGF, etc.), Noggin, and Noggin-conditioned media.

In the present specification, the term "organoids" may refer to cell aggregates having an average diameter of about 30 µm or more, about 40 µm or more, about 50 µm or more, about 60 µm or more, about 70 µm or more, about 80 µm or more, about 90 µm or more, or about 100 µm or more. As the size of the organoids increases, drug delivery into the organoids is limited, and the responsiveness of the organoid to the drug is limited. In addition, as the maximum value of the average diameter of the collected organoids increases, the difference in size (average diameter) between the organoids included increases, so that the difference in responsiveness to the same drug between the organoids increases. Thus, in order to obtain a uniform drug responsiveness result, it is important to determine the upper limit of the size (average diameter) of the organoids. The present disclosure has a remarkable effect of increasing the accuracy of the drug responsiveness analysis result by applying mechanical stress to the organoids and post-treating the organoids in two steps to uniformize the size of the obtained organoids. This process is expected to be available to obtain a uniform yield when culturing organoids derived from the pancreas as well as organoids derived from other tissues.

Therefore, in order to obtain a uniform result by increasing the degree to which the finally obtained organoids simulate *in vivo* drug responsiveness and minimizing the difference in drug responsiveness between the organoids, step (2) of collecting the organoids may be characterized by selectively collecting organoids having a predetermined average diameter or less, for example, 1000 µm or less, 900 µm or less, 800 µm or less, 700 µm or less, 600 µm or less, 500 µm or less, 400 µm or less, 300 µm or less, or 200 µm. The organoids collected in step (2) may have an average diameter of 30 to 1,000 µm, 40 to 1,000 µm, 50 to 1,000 µm, 60 to 1,000 µm, 70 to 1,000 µm, 80 to 1,000 µm, 90 to 1,000 µm, 100 to 1,000 µm, 30 to 900 µm, 40 to 900 µm, 50 to 900 µm, 60 to 900 µm, 70 to 900 µm, 80 to 900 µm, 90 to 900 µm, 100 to 900 µm, 30 to 800 µm, 40 to 800 µm, 50 to 800 µm, 60 to 800 µm, 70 to 800 µm, 80 to 800 µm, 90 to 800 µm, 100 to 800 µm, 30 to 700 µm, 40 to 700 µm, 50 to 700 µm, 60 to 700 µm, 70 to 700 µm, 80 to 700 µm, 90 to 700 µm, 100 to 700 µm, 30 to 600 µm, 40 to 600 µm, 50 to 600 µm, 60 to 600 µm, 70 to 600 µm, 80 to 600 µm, 90 to 600 µm, 100 to 600 µm, 30 to 500 µm, 40 to 500 µm, 50 to 500 µm, 60 to 500 µm, 70 to 500 µm, 80 to 500 µm, 90 to 500 µm, 100 to 500 µm, 30 to 400 µm, 40 to 400 µm, 50 to 400 µm, 60 to 400 µm, 70 to 400 µm, 80 to 400 µm, 90 to 400 µm, 100 to 400 µm, 30 to 300 µm, 40 to 300 µm, 50 to 300 µm, 60 to 300 µm, 70 to 300 µm, 80 to 300 µm, 90 to 300 µm, 100 to 300 µm, 30 to 200 µm, 40 to 200 µm, 50 to 200 µm, 60 to 200 µm, 70 to 200 µm, 80 to 200 µm, 90 to 200 µm, or 100 to 200 µm.

Step (2) of collecting organoids may be performed by any conventional means that may be used to select organoids having a predetermined size (an average diameter of 1000 µm, 900 µm, 800 µm, 700 µm, 600 µm, 500 µm, 400 µm, 300 µm, or 200 µm) or less. For example, step (2) of collecting organoids may include a step of collecting organoids that passed through a cell strainer having a pore size of 1000 µm, 900 µm, 800 µm, 700 µm, 600 µm, 500 µm, 400 µm, 300 µm, or 200 µm.

Step (3) of allowing organoids to grow may include a step of seeding the organoids at a confluency of 30% (v/v) to 95% (v/v), 30% (v/v) to about 90% (v/v), 30% (v/v) to about 85% (v/v), 30% (v/v) to about 80% (v/v), 30% (v/v) to about 75% (v/v), 30% (v/v) to about 70% (v/v), 40% (v/v) to 95%(v/v), 40% (v/v) to about 90% (v/v), 40% (v/v) to about 85% (v/v), 40% (v/v) to about 80% (v/v), 40% (v/v) to about 75% (v/v), 40% (v/v) to about 70% (v/v), 50% (v/v) to 95% (v/v), 50% (v/v) to about 90% (v/v), 50% (v/v) to about 85% (v/v), 50% (v/v) to about 80% (v/v), 50% (v/v) to about 75% (v/v), or 50% (v/v) to about 70% (v/v)), and allowing the seeded organoids to grow for 12 to 36 hours, 12 to 30 hours, 12 to 25 hours, 18 to 36 hours, 18 to 30 hours, 18 to 24 hours, 23 to 36 hours, 23 to 30 hours, or 23 to 25 hours. Only when the distribution of the seeded organoids is uniform, it is possible or easy to control the growth of the organoids, and a drug may be delivered into the organoids at a uniform level when the organoids are treated with the drug. For this reason, the confluency represented by the following equation may advantageously be controlled within a predetermined range: Volume proportion (%(v/v)) of organoids in organoid growth medium) = [(organoid volume)/(organoid volume + medium volume)] * 100). If the confluency is lower than 30% (v/v), the interaction between the organoids may be insufficient, and thus the growth of the organoids may be adversely affected, and if the confluency is higher than 95%, the density of the organoids in the container may increase, and thus the contact area between each organoid and the medium may decrease, so that not only the growth of the organoids but also the drug delivery rate during drug treatment using the medium may be adversely affected. Accordingly, the confluency of the organoids in step (3) of allowing organoids to grow is preferably controlled within the above-described range. The method may include a step of collecting a portion of the organoid tissue obtained through the culture and applying mechanical stress thereto.

Still yet further another example provides a composition or kit for measurement (prediction or verification) of drug responsiveness or for drug screening, the composition or kit containing an organoid prepared by the method for producing an organoid for use in measurement of drug responsiveness or in drug screening.

Another example provides a method for measurement (prediction or verification) of drug responsiveness or for drug screening, the method including a step of treating organoids, prepared by the method for producing organoids for use in measurement of drug responsiveness or in drug screening, with the drug. The method may further include steps (1) to (3) of the above-described method for producing organoids, before treating the organoids with the drug. The method may further include the comparing step and/or determining step of the above-described method for verifying drug effect.

The composition or kit or method for use in measurement (prediction or verification) of drug responsiveness or for drug screening, which is provided in the present specification, may be a standardized technology that is universally applicable to all types of organoids regardless of the origin, form, etc. of the organoid.

In one embodiment of the present disclosure, there is provided a cancer animal model with deletion of telomerase. In the cancer animal model, the deletion of telomerase may be induced by knockout of telomerase RNA component. A BRCA2 gene in the cancer animal model may be mutated. In the cancer animal model, the cancer may be pancreatic cancer.

In another embodiment of the present disclosure, there are provided cancer organoids produced from the animal model. The cancer organoids may be pancreatic cancer organoids.

In still another embodiment of the present disclosure, there is provided a method for producing cancer organoids, the method including steps of: (a) isolating tissue from the animal model; and (b) mixing cells, isolated from the tissue, with Matrigel, and culturing the mixed cells to form pre-organoids. In the method, the tissue may be pancreatic tissue. The method may further include, after step (b), step (c) of dissociating the pre-organoids with a mechanical stress of 50 to 500 Pa, and (d) re-culturing the dissociated organoids. In the method, the cancer organoids have a diameter of 100 to 200 µm.

In yet another embodiment of the present disclosure, there is provided a method for verifying the effect of a candidate drug for cancer treatment, the method including steps of: (a) producing cancer organoids by any one of the above-described methods; (b) treating the organoids with the candidate drug for cancer treatment; and (c) measuring the cell viability of the organoids. In the method, the cancer may be pancreatic cancer, and the drug may be any one or more selected from the group consisting of histone deacetylase inhibitors (HDACi), PARP-1 (poly[ADP-ribose]polymerase 1) inhibitors, and plk1 (polo-like kinase 1) inhibitors.

In still yet another embodiment of the present disclosure, there is provided a method for verifying responsiveness to a candidate drug for cancer treatment, the method including steps of: (a) producing cancer organoids by any one of the above-described methods; (b) treating the organoids with the candidate drug for cancer treatment; and (c) identifying expression of a biomarker that binds specifically to either a nucleic acid encoding the tumor suppressor gene p53, or a protein synthesized therefrom. In the method, the cancer may be pancreatic cancer, and the drug may be any one or more selected from the group consisting of histone deacetylase inhibitors (HDACi), PARP-1 (poly[ADP-ribose]polymerase 1) inhibitors, and plk1 (polo-like kinase 1) inhibitors.

More specifically, the biomarker is preferably a primer or probe that binds specifically to a nucleic acid encoding the tumor suppressor gene p53, or the biomarker is preferably an antibody that binds specifically to a p53 protein synthesized from a nucleic acid encoding the tumor suppressor gene p53.

Hereinafter, each step of the present disclosure will be described in detail.

### Advantageous Effects

The present disclosure provides: organoids derived from a BubR1^{K243R/+} mouse in which the 243^{rd} lysine in one allele of BubR1 has been substituted with arginine; organoids derived from a Brca2^{F11/F11} mouse in which Brca2 exon 11 has been knocked out by conditional treatment with tamoxifen; organoids derived from a mTR^{-/-} mouse in which telomerase reverse transcriptase RNA component (Terc, mTR) genes have been knocked out in the same manner; and organoids derived from a Brca2^{F11/F11};mTK^{-/-}mouse obtained by crossing of mice having the above-described two genotypes. These organoids may be advantageously applied to studies, including prediction of drug efficacy before drug treatment, verification of drug efficacy (effect and responsiveness) after drug treatment, karyotyping depending on genotype, and observation of division rate and pattern. The present disclosure also provides technology for identification of drug responsiveness before and after standardized drug treatment, which is universally applicable to various organoids, and technology for drug screening.

### Brief Description of Drawings

FIG. 1 depicts images showing the results of observation with an inverted microscope (Zeiss) for the cultured state of pancreatic organoids derived from mutant mice with deletion of BRCA2 and/or TERC gene.
FIG. 2a depicts images showing the results of gel electrophoresis performed to identify gene mutations (BRCA2 gene exon 11 deletion (Brca2^{F11/F11}), TERC gene knockout (mTR^{-/-}), and CreER^{™} gene insertion (CreER^{™})) in organoids (M: DNA marker for band size identification, DW (distilled water): PCR negative control, WT: wild-type, Brca2^{F11/F11}: band showing Brca2 exon 11 flanked by loxP, mTR^{-/-}: band showing TERC gene knockout, and Cre-ER^{™}: band showing insertion of Cre recombinase gene).
FIG. 2b shows gel electrophoresis results showing PCR products depending on whether or not the organoids shown to have the BRCA2^{F11/F11} genotype in FIG. 2a are treated with 4-OHT. Here, (-) shows the result obtained when the organoids were not treated with 4-OHT, and (+) shows the result obtained when the organoids were treated with 4-OHT to induce deletion of BRCA2 gene exon 11.
FIG. 3 depicts images showing the results of observation with an inverted microscope (Zeiss) for the cultured state of pancreatic organoids derived from mutant mice into which a BubR1 gene with a mutation of K243R has been inserted.
FIG. 4 is a photograph showing the results of gel electrophoresis performed to confirm whether or not a BubR1 gene with a mutation of K243R has bene inserted in pancreatic organoids derived from a genetically mutated mouse (BubR1^{K243R/+}) (M: DNA marker for band size classification, DW (distilled water): PCR negative control).
FIG. 5a is a photograph showing the results of gel electrophoresis performed to identify organoids (not treated with 4-OHT) having a normal BRCA2 gene and organoids (treated with 4-OHT) having a partial deletion of BRCA2.
FIG. 5b depicts photographs showing that pancreatic organoids derived from BRCA2 gene-deleted mice (Brca2^{F11/F11} CreER^{™}) have been treated with a histone deacetylase inhibitor (HDACi).
FIG. 6 depicts photographs showing that pancreatic organoids derived from BRCA2 gene-deleted mice (Brca2^{F11/F11}; CreER^{™}) have been treated with a PARP-1 inhibitor or a plk1 inhibitor alone or in combination with a histone deacetylase inhibitor (HDACi).
FIG. 7 shows the results of observing pancreatic mouse organoids having a wild-type gene for 24 hours after treatment with 1 µM TSA (trichostatin A), through viable cell tracking using the IncuCyte S3 Live-Cell Analysis System (Essen bioscience) (scale bar: 2.1 mm).
FIG. 8 depicts photographs showing the degree of growth of organoids depending on whether or not pancreatic organoids from Brca2^{F11/F11}; mTR^{-/-}; Cre-ER^{™} mice obtained through three generations (G3) by crossing have been treated with 4-hydroxytamoxifen (4-OHT).
FIG. 9 depicts fluorescence images showing the results of immunofluorescence analysis of pancreatic organoids derived from BubR1^{K243R/+} mouse. The left image shows the results obtained for pancreatic organoids derived from a wild-type mouse, and the right image shows the results obtained for pancreatic organoids derived from BubR1^{K243R/+} mice.
FIG. 10 depicts micrographs showing the drug responsiveness of BubR1^{K243R/+} mouse pancreatic organoids to AR-42 (top) and ACY-241 (bottom).
FIG. 11 is a schematic view showing the structure of mouse BubR1 protein.
FIG. 12 shows the results of comparing the size uniformity of organoids depending on whether an organoid culture step further includes a step of physically dissociating organoids by application of mechanical stress.
FIG. 13 depicts photographs showing the results of observation with an inverted microscope (Zeiss) on day 5 after pancreatic organoids from K-ras^{G12D} mutant mice and wild-type organoids were each treated with 100 µM, 200 µM or 500 µM of Resveratrone drug.
FIG. 14a shows the results of identifying GFP after pancreatic organoids from K-ras^{G12D} mutant mice were treated with 0 µM, 200 µM or 500 µM of Resveratrone drug.
FIG. 14b shows the results of measuring organoid viability by treatment with each of a TdT enzyme-containing staining solution and a DAPI reagent using a TUNEL assay on day 5 after pancreatic organoids from K-ras^{G12D} mutant mice were treated with 0 µM, 200 µM or 500 µM of Resveratrone drug.
FIG. 14c shows the results of measuring organoid viability by treatment with a staining solution using an MTT assay on day 5 after pancreatic organoids from K-ras^{G12D} mutant mice were treated with 0 µM, 200 µM or 500 µM of Resveratrone drug.
FIG. 14d graphically shows the results of measuring organoid viability by an MTT assay on day 5 after pancreatic organoids from K-ras^{G12D} mutant mice were treated with 0 µM, 200 µM or 500 µM of Resveratrone drug.
FIG. 15 shows the results of analyzing the expression level of p53 by a Western blot technique to examine responsiveness to Resveratrone drug after mouse-derived or patient-derived pancreatic organoids were treated with the drug.

### Best Mode for Carrying Out the Disclosure

Conventional two-dimensional cell culture methods have a limitation in that information on derived cancer cell tissue does not accurately reflect an *in vivo* situation, and conventional organoid drug responsiveness test methods have a technical limitation in that uniform drug treatment is not achieved, resulting in poor accuracy in verifying drug effect. The present disclosure have been made in order to overcome these limitations and is intended to perform anticancer therapeutic research more efficiently and furthermore, to verify drug effect in a uniform and accurate manner. The present disclosure is intended to model cancer closely to *in vivo* cancer using organoids obtained from normal tissue and cancer tissue of a genetically mutated mouse, and to provide a method of producing uniform organoids from pre-organoids through a post-treatment process in order to usefully apply these organoids to the verification of drug efficacy depending on genetic mutation.

### Mode for Carrying Out the Disclosure

The present disclosure will be described in more detail below with reference to examples, but these examples are for illustrative purposes only and are not intended to limit the scope of the present disclosure. It will be obvious to those skilled in the art that the examples described below may be modified without departing from the essential subject matter of the present disclosure.

### Example 1: Generation of Pancreatic Organoids Derived from Genetically Mutated Mice

The following mice were prepared: mice (Brca2^{F11F11}; exon 11 deletion) in which BRCA2 gene exon 11 is flanked by loxP through Cre-loxP recombination using bacteriophage P1 and induction of conditional BRCA2 deletion using CreER recombinase is possible (Jos Jonkers, Ralph Meuwissen, Hanneke van der Gulden, Hans Peterse, Martin van der Valk and Anton Berns. 2001. Synergistic tumor suppressor activity of BRCA2 and p53 in a conditional mouse model for breast cancer. Nature Genetics. Vol. 29, pages 418-425); mice (mTR^{-/-}) lacking telomerase activity due to knockout of telomerase RNA component (TERC) gene; and CMV-Cre mice (CreER^{™}) with a CMV promoter.

### 1.1. Construction of BRCA2 Exon 11-Targeting Vector

To target the BRCA2 locus, a 13.5-kb λ phage clone including mouse BRCA2 gene (NM_001081001.2) exons 8 to 12 was isolated from a genomic 129/Sv library (Agilent technologies). The phage insert was excised with NotI and subcloned in pGEM5 (Promega). Then, a loxP-PGKneor-PGKtk-loxP dual selection cassette (Thermo Fisher Scientific) was inserted into an AvrII site in mouse BRCA2 gene intron 11, and a single loxP site was inserted into a NspV site in mouse BRCA2 gene intron 10 in a direct orientation relative to the floxed selection cassette.

### 1.2. Construction of Telomerase RNA Component (TERC) Gene-Targeting Vector

Mice with a complete deletion of telomerase RNA component (TERC) gene (GenBank Accession No. NR_001579.1) were obtained from Jackson Lab (Stock No: 004132, B6.Cg-Terctm1Rdp/J) and used in the following experiment.

### 1.3. Generation of Mice (Brca2^{F11/11};CreER^{™}) with Deletion of BRCA2 Exon 11

The vector prepared in Example 1.1 above was separated, purified, and electroporated into 129/Ola-derived mouse ES cells of the E14 subclone IB10 (The Netherlands Cancer Institute). Colonies were screened by Southern blot analysis for correct insertion of the floxed selection marker and the single loxP site. The embryonic stem cells were electroporated with a mixture of the Cre expression plasmid pOG231 (Addgene) and PGKpuro (Addgene) in a molar ratio of 10:1 to induce transient Cre recombinase activity and resistance to puromycin. 20 hours after electroporation, puromycin (1.8 µg/ml) was added to medium and incubated for 48 hours. Then, dead cells were removed and surviving embryonic stem cells were cultured for additional 10 days in nonselective medium. The resulting embryonic stem cell clones were analyzed by Southern blotting to detect successful deletion of the floxed marker, insertion of a single loxP site and generation of the conditional allele.

Germline chimeras were generated by injecting 12 to 15 mutant 129/Ola embryonic stem cells obtained as described above into C57Bl/6 blastocysts and were crossed with FVB/N mice (Jackson Laboratory) to produce outbred heterozygous offspring (BRCA2 conditional mutant). The obtained BRCA2 conditional mutants were crossed with Cre mice (CMV-Cre mice (CreER^{™}) with a CMV promoter; Jackson Laboratory, Stock No. 004847), and Cre-mediated deletion of floxed alleles in the germline was performed to generate BRCA2 conditional deletion mice (Brca2^{F11/F11};CreER^{™} or Brca2^{F11/F11} indicated by mTR^{+/+};CreER^{™}).

### 1.4. Generation of BRCA2 exon 11-deleted and TERC gene-knockout mice (Brca2^{F11/F11};mTK^{-/-};CreER^{™})

As described in Example 1.3 above, Brca2^{F11/F11} CreER^{™} mice were obtained from offspring produced by crossing Brca2^{F11/F11} mice with CreER^{™} mice, and Brca2^{F11/F11};CreER^{™}; mTK^{-/+} mice were obtained from offspring produced by crossing the Brca2^{F11/F11};CreER^{™} mice with mTR^{-/+} mice (Example 1.2), and these mice were crossed to obtain Brca2^{F11/F11}; CreER^{™};mTR^{-/-} mice with Mendelian probability.

### 1.5. Generation of mice (BubR1^{K243R/+}) into which BubR1 gene with K243R Mutation has been inserted

Through site-directed mutagenesis, the BubR1 gene mutated by inducing lysine (K)-to-arginine (R) (K243R) at position 243 of BubR1 (GenBank NP_033903.2; coding gene: NM_009773.3) was inserted into 129/Sv embryonic stem cells (Agilent Technologies) using a pBluescript KS(+) vector (Addgene), and the cells were injected into C57BL/6 mouse blastocysts to obtain heterozygous mutant mice (BubR1^{K243R/+}) into which a mutant gene encoding BubR1 with K243R mutation has been inserted (see Inai Park et al. 2013. Loss of BubR1 acetylation causes defects in spindle assembly checkpoint signaling and promotes tumor formation. Journal of Cell Biology. 202 (2):295).

### Example 2: Generation of Pancreatic Organoids Derived from Mutant Mice with Deletion of BRCA2 and/or TERC Gene

From each of the mutant mice generated in Example 1.3 and Example 1.4, pancreatic organoids were generated in the following manner.

Each mouse was euthanized and then dissected to obtain pancreatic tissue. As a dissociation solution, a mixture of 3 to 5 mml of HBSS (Hank's Balanced Salt Solution; GIBCO^{®}), 1 mg/ml of collagenase P (Roche) and 0.1 mg/ml of DNase 1 (Sigma Aldrich) was prepared by warming in a water bath at 37°C. The obtained entire mouse pancreatic tissue (Vpan=1.08 mg/mm³) was transferred into a 100-mm Petri dish. Then, 100 µl of the prepared dissociation solution was added thereto, and the tissue was finely cut 5 to 10 times using scissors or a knife. The finely cut pancreatic tissue (Vpan=1.08 mg/mm³) was transferred into a 50-ml conical tube, and 3 to 5 ml of the dissolution solution was added thereto. The tube was incubated in a shaking incubator at 230 rpm and 37°C. After 10 to 15 minutes, the tube was taken out and 10 to 15 ml of cold FBS was added thereto. The obtained dissociated pancreatic tissue was passed through a cell strainer having a pore size of 100 µm and washed 2 to 3 times with HBSS. The pancreatic tissue, which did not pass through the cell strainer, was observed under a microscope, and ductal cells were picked and selected. A process of centrifuging the selected ductal cells at 1500 rpm for 10 minutes to collect pellets and then washing the pellets with HBSS was repeated twice.

The obtained cell pellets and 200 µl of Matrigel (Corning) were mixed together so that a volume ratio of the cell pellets and the Matrigel was 1:5, and then the mixture was seeded into a 12-well or 24-well plate in an amount of 100 to 150 µl per well in the case of the 12-well plate. About one hour after cell seeding, when the Matrigel hardened, a medium was added to the 12-well plate in an amount of 1 ml or to the 24-well plate in an amount of 500 µl, and incubated in an incubator at 37°C under 10% CO₂ for 48 to 72 hours or longer. The medium used at this time had the following composition: A mixture of DMEM/F-12 (Dulbecco's Modified Eagle Medium/Ham's F-12; Thermo Fisher Scientific) with 1% (vol/vol) penicillin/streptomycin, 10 mM HEPES, 1% GlutaMAX, 1:50 B27 supplement (Gibco), 1 mM N-acetylcysteine, 5% (vol/vol) Rspo 1-conditioned medium (Hans Clevers lab), 10 mM nicotinamide, 10 nM recombinant human [Leu15]-gastrin I (Sigma Aldrich), 50 ng/ml of recombinant mouse EGF (Peptron), 100 ng/ml of recombinant human FGF10 (Peptron), and 25 ng/ml of recombinant human Noggin (Peptron) or 5% (vol/vol) Noggin-conditioned medium (Hans Clevers lab).

In order to induce deletion of the BRCA2 gene and/or the TERC gene, the organoids were treated with 4-hydroxytamoxifen (4-OHT). Specifically, 4-hydroxytamoxifen (4-OHT) was dissolved in the organoid culture at 400 nM and incubated for 3 weeks or longer, starting from a time point, at which 24 to 48 hours lapsed after the pancreas was initially isolated from the mice and then culture thereof into organoid was constructed, until immediately before a time point at which a drug-treatment experiment was performed for drug screening as described below (see Examples 4 and 5).

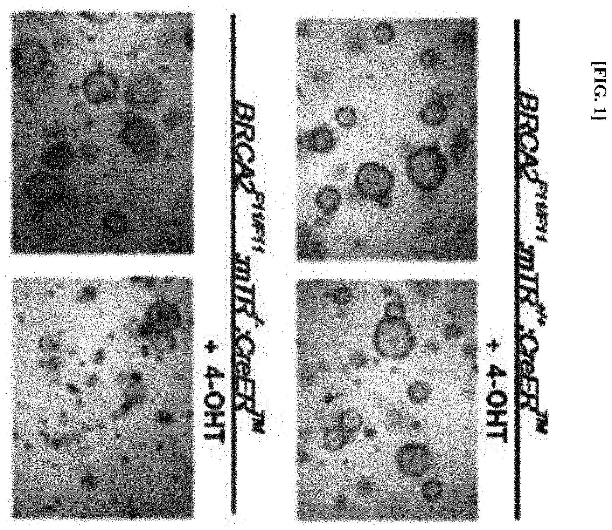

The results of observing the above-described pancreatic organoid culture with an inverted microscope (Zeiss) are shown in FIG. 1. As shown in FIG. 1, it can be confirmed that pancreatic organoids were successfully produced regardless of whether the BRCA2 gene and/or the TERC gene was deleted (whether treatment with 4-OHT was performed). It can be confirmed that the BRCA2 gene-deleted organoids were somewhat small in shape, but grew in a similar pattern to a wild type as the culture gradually proceeded.

The genotype of the organoids was identified by performing genomic PCR DNA gel electrophoresis on the genomic DNA extracted by lysis of the obtained pancreatic organoids. Specifically, PCR was performed for 30 cycles, each consisting of denaturation at 95°C for 1 min, annealing at 55°C for 30 sec, and elongation at 72°C for 1 min. For electrophoresis, 5 µl of the PCR product was mixed with 5 µl of bromophenol blue or xylene on 1% (w/v) agarose gel and electrophoresed at 100 mV. The primers used in the PCR are as follows:

**[Table 1]**

| Primer | Nucleic acid sequence (5'→3') | SEQ ID NO |
|---|---|---|
| Brca2-11F | CTCATCATTTGTTGCCTCACTTC | 1 |
| Brca2-11R | TGTTGGATACAAGGCATGTAC | 2 |
| mTR-WT | GCACTCCTTACAAGGGACGA | 3 |
| mTR-common | CTTCAATTTCCTTGGCTTCG | 4 |
| mTR-mutant | ATTTGTCACGTCCTGCACGACG | 5 |
| CRE-3F | CGGCATGGTGCAAGTTGAAT | 6 |
| CRE-3R | CGGTGCTAACCAGCGTTTTC | 7 |
| CRE-internal-F | CTAGGCCACAGAATTGAAAGATCT | 8 |
| CRE-internal-R | GTAGGTGGAAATTCTAGCATCATCC | 9 |

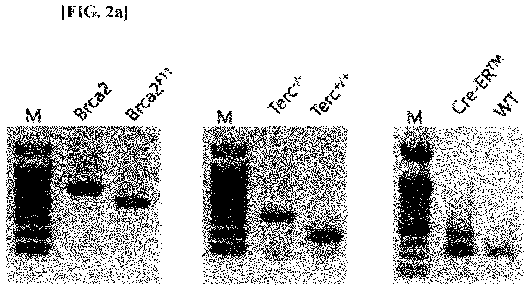

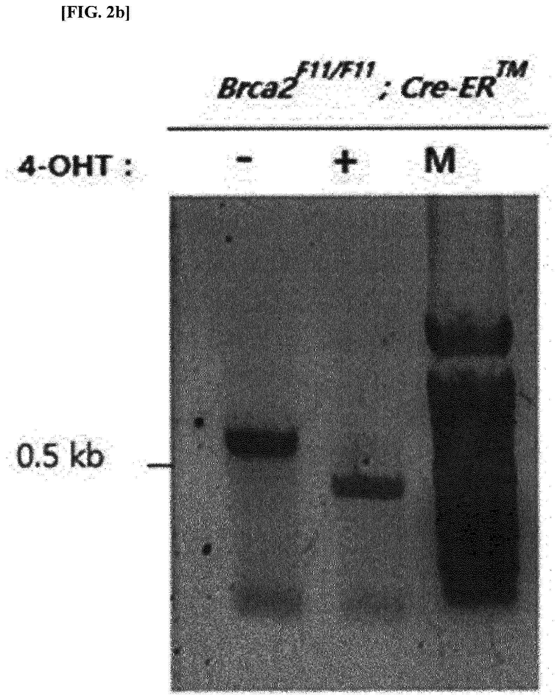

The obtained results are shown in FIGS. 2a and 2b.

FIG. 2a depicts images showing the results of gel electrophoresis performed to confirm genetic mutations (deletion of BRCA2 gene exon 11) (Brca2^{F11/F11}), knockout of TERC gene (mTR^{-/-}), and insertion of CreER^{™} gene (CreER^{™}) in organoids (M: DNA marker for band size identification, DW: PCR negative control, WT: wild-type, Brca2^{F11/F11}: band showing Brca2 exon 11 flanked by loxP, mTR^{-/-}: band showing TERC gene knockout, CreER^{™}: band showing insertion of the Cre recombinase gene).

FIG. 2b shows gel electrophoresis results showing PCR products depending on whether or not the organoids confirmed to have the BRCA2^{F11/F11} genotype in FIG. 2a are treated with 4-OHT. Here, (-) shows the result obtained when the organoids were not treated with 4-OHT, and (+) shows the result obtained when the organoids were treated with 4-OHT to induce deletion of BRCA2 gene exon 11. As shown in FIG. 2b, it can be confirmed that, when the organoids were treated with 4-OHT, the length of the PCR product became shorter than when the organoids were not treated with 4-OHT, indicating that BRCA2 gene exon 11 was deleted.

### Example 3: Generation of Pancreatic Organoids Derived from Heterologous Mutant Mice into Which BubR1 Gene with K243R Mutation Has Been Inserted

With reference to the method of Example 2, pancreatic organoids were produced from the heterozygous mutant mice (BubR1^{K243R/+}) which were produced in Example 1.5 above and in which the mutant BubR1 gene with induced K243R mutation has been knocked in. For comparison, pancreatic organoids derived from wild-type mice were generated with reference to Example 2.

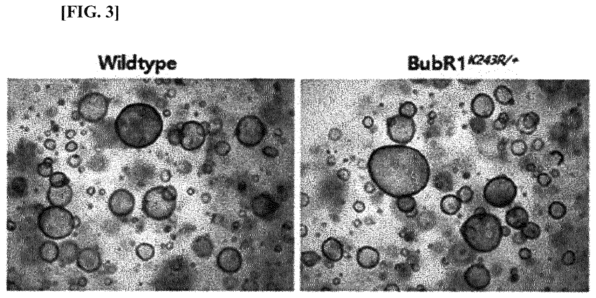

The results of observing the obtained pancreatic organoids with an inverted microscope (Zeiss) are shown in FIG. 3. As shown in FIG. 3, it can be confirmed that pancreatic organoids could be successfully produced from the mutant mice (BubR1^{K243R/+}), like the case of the wild-type mice.

The genotype of the organoids was identified by performing genomic PCR DNA gel electrophoresis on the genomic DNA extracted by lysis of the obtained pancreatic organoids. Specifically, PCR was performed for 30 cycles, each consisting of denaturation at 95°C for 1 min, annealing at 55°C for 30 sec, and elongation at 72°C for 1 min. For electrophoresis, 5 µl of the PCR product was mixed with 5 µl of bromophenol blue or xylene on 1% (w/v) agarose gel and electrophoresed at 100 mV. The primers used in the PCR were as follows:

**[Table 2]**

| Primer | Nucleic acid sequence (5'→3') | SEQ ID NO |
|---|---|---|
| BubR1K243R/+ F | GAGGTAAAGGCAGGGGAATC | 10 |
| BubR1K243R/+ R | GAGAAAGCGGGGGTCATTAT | 11 |

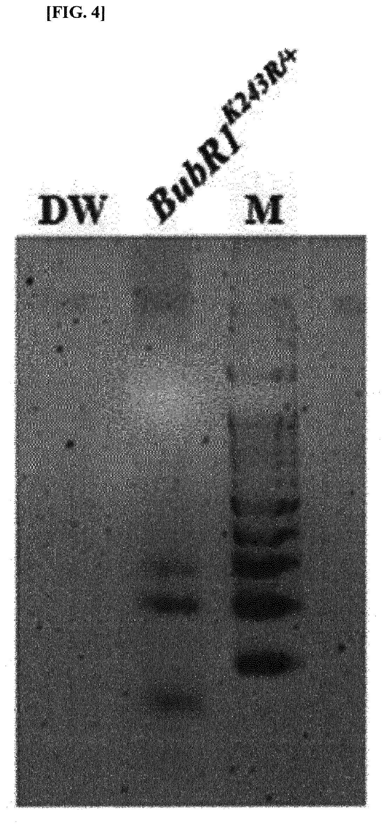

The obtained results are shown in FIG. 4.

FIG. 4 is an image showing the results of gel electrophoresis performed to confirm whether or not a BubR1 gene with a mutation of K243R has bene inserted in pancreatic organoids derived from a genetically mutated mouse (BubR1^{K243R/+}) (M: DNA marker for band size identification, DW: PCR negative control). As shown in the lane indicated by BubR1^{K243R/+} in FIG. 4, bands at 145 bp and 219 bp were observed, indicating that knock-in of the gene of interest has successfully occurred.

### Example 4: Test for Drug Responsiveness of Pancreatic Organoids Derived from Mutant Mice

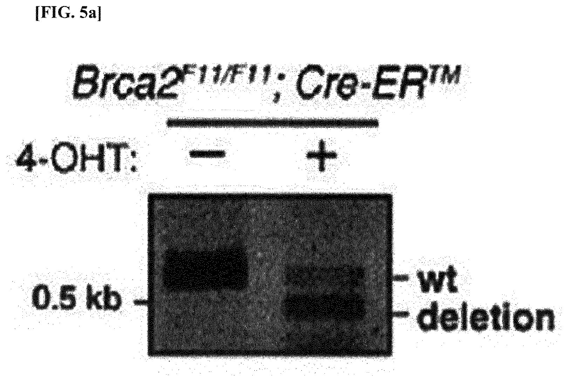

With reference to the method of Example 2 above, pancreatic organoids derived from BRCA2 gene-deleted mice (Brca2^{F11/F11};CreER^{™}) were prepared. To induce a partial deletion of BRCA2 gene (deletion of exon 11 of BRCA2 gene), 4-hydroxytamoxifen (4-OHT) was added to the organoids in an amount of 400 nM, and the resulting organoids were cultured for 3 weeks or more (see Example 2). For comparison, organoids not treated with 4-OHT (in which BRCA2 gene is normal) were also prepared. FIG. 5a is a photograph showing the results of gel electrophoresis performed to confirm organoids (not treated with 4-OHT) having normal BRCA2 gene and organoids (treated with 4-OHT) with a partial deletion of BRCA2.

Organoids having normal BRCA2 gene (not treated with 4-OHT) and organoids with a partial deletion of BRCA2 (exon 11 deletion) were seeded in equal amounts into 24-well plates. On the day after seeding, the organoids were treated with a mixture of the anticancer agent histone deacetylase inhibitor (HDACi) and a culture medium (see Example 2). The types and treatment concentrations of HDACi used at this time are shown in Table 3 below:

**[Table 3]**

| Type of HDACi | Treatment concentration |
|---|---|
| Trichostatin A (TSA) | 1 µM |
| Suberoylanilide hydroxamic acid (SAHA) | 17 µM |
| LMK-235 | 3 µM |
| FK-228, Romidepsin | 10 nM |

At this time, treatment with HDACi and treatment with 4-OHT were not performed simultaneously for pancreatic organoids in which BRCA2 had already been knocked out.

3 days after treatment (first treatment) with HDACi, the culture medium was replaced with a fresh medium, and treatment (second treatment) with each HDACi was performed again at the concentration shown in Table 3. Here, the primary treatment with HDACi was performed about 24 to 48 hours after seeding of the organoids, and 3 days after that, the second treatment was performed. On 6 days after the first treatment with HDACi, the state of the organoids treated with HDACi was observed using an inverted microscope.

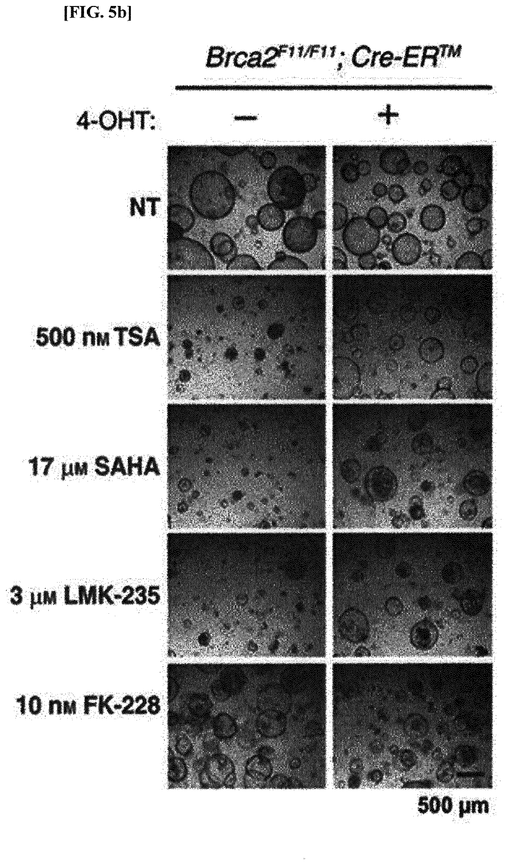

The obtained results are shown in FIG. 5b. As shown in FIG. 5b, it was observed that responsiveness to the tested drugs did differ depending on the presence or absence of the BRCA2 gene. Thereby, it can be confirmed that the pancreatic organoids from the Brca2^{F11/F11} mice can be effectively used for verification of the effect of a drug associated with the BRCA2 gene and/or for drug screening.

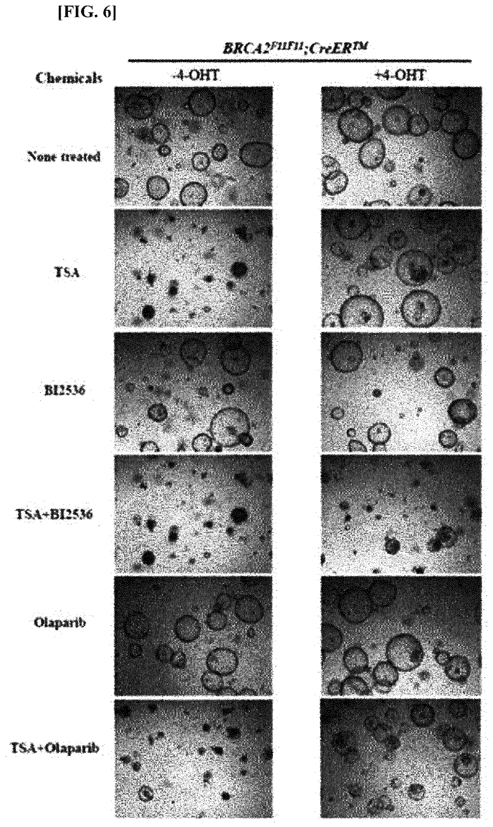

In addition, using the above-described method, the state of the organoids treated with the PARP-1 inhibitor Olaparib (10 µM) or the plk 1 inhibitor BI2536 (10 nM) alone or in combination with HDACi (200 nM TSA) was observed, and the results are shown in FIG. 6. As shown in FIG. 6, it was observed that responsiveness to the tested drugs did differ depending on the presence or absence of the BRCA2 gene. Thereby, it can be confirmed that the pancreatic organoids from the Brca2^{F11/F11} mice can be effectively used for verification of the effect of a drug associated with the BRCA2 gene and/or for drug screening.

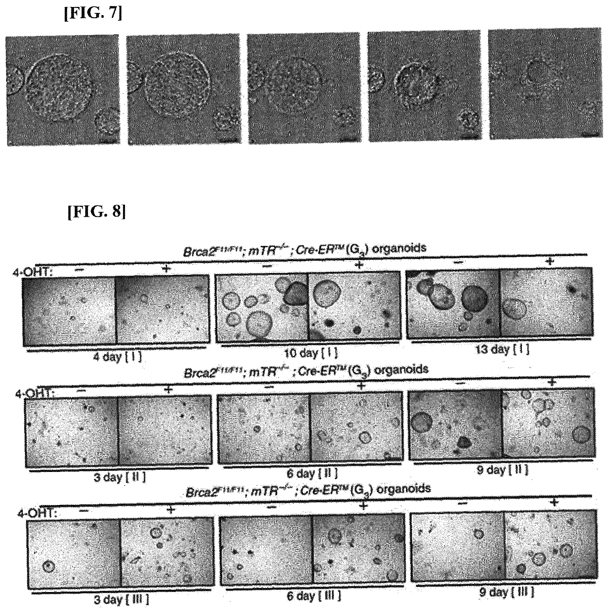

FIG. 7 shows the results of observing pancreatic mouse organoids having a wild-type gene for 24 hours after treatment with 1 µM TSA (trichostatin A), through viable cell tracking using the IncuCyte S3 Live-Cell Analysis System (Essen bioscience). Respective images are images taken at 4-hour intervals. Scale bar represents 2.1 mm.

### Example 5. Construction of Telomerase Knockout Cancer Model (TKCM) by Continuous Culture of Brca2^{F11/F11};mTK^{-/-};Cre-ER^{™} Mice Pancreatic Organoids

Using the pancreatic organoids from Brca2^{F11/F11};mTR^{-/-};Cre-ER^{™} generation 3 (G3) mice obtained through three generations by crossing of Brca2^{F11/F11};mTR^{-/-};Cre-ER^{™} mice, a telomerase-knockout cancer model (TKCM) was constructed, which continues to divide by maintaining the telomere length even in the absence of telomerase. In order to overcome the disadvantages of two-dimensionally cultured carcinogenic cell lines used in conventional carcinogenesis inducing mechanism studies, that is, disadvantages that genomic mutations have already accumulated and all *in vivo* environmental characteristics are not reflected, TKCM mice (G3) were generated through three-way crossing of Brca2^{F11/F11};mTR-/-;Cre-ER^{™} mice. In addition, pancreas-derived organoids were generated, which can simulate an in-vivo environment of the organ in the mice of interest and allows direct identification of whether growth is inhibited, and it was confirmed that actual cancer was induced.

More specifically, for generation of the mouse pancreatic organoid cancer model, with reference to Example 2, pancreatic organoids were generated from Brca2^{F11/F11}; mTR^{-/-}; Cre-ER^{™} mice obtained through three generations (G3) by crossing. Then, the organoids were treated or not treated with tamoxifen (4-OHT) so that Brca2 deficiency was induced or not induced. The treatment concentration of 4-OHT was set to 400 nM. The pancreatic organoids were treated with 4-OHT once every 2 days during a time period ranging from one day after seeding of the pancreatic organoids into plates to before treatment with a drug. Then, the organoids were observed with an inverted microscope (Zeiss) once every three days, and the results are shown in FIG. 8. The organoids were subcultured on day 10 in the order of [I], [II], and [III] shown in FIG. 8, and the degree of growth thereof was compared.

In [I], it was confirmed that the organoids (+4-OHT) deficient in both BRCA2 and TERC exhibited somewhat slower growth than the organoids (-4-OHT) deficient in only TERC.

In [II] (comparison of the degree of growth of organoids mechanically separated from organoids of [I]), it was confirmed that the growth of the organoids (-4-OHT) deficient in only TERC was inhibited compared to that at the previous passage, and that the growth of the organoids (+4-OHT) deficient in both BRCA2 and TERC was slower than that at the previous passage.

In [III] (comparison of the degree of growth of organoids mechanically separated from organoids of [II]), it was confirmed that the growth of the organoids (-4-OHT) deficient in only TERC was greatly inhibited so that sustainable culture was no longer possible, whereas the organoids (+4-OHT) deficient in both BRCA2 and TERC were in a state where sustainable culture was possible. These results confirm that organoids were cultured in which the carcinogenic mechanism was activated and carcinogenesis proceeded.

### Example 6. Imaging of BubR1^{K243R/+} Mouse Pancreatic Organoids through Immunofluorescence Assay (IFA)

With reference to Examples 2 and 3, the BubR1^{K243R/+} mouse pancreatic organoids were cultured, and then stained with DAPI (blue), Tubulin (green) and BubR1 (red). As a result, it was confirmed that the generated organoids can be utilized as a new model to study genome instability.

First, the cultured organoids were transferred into a 15-ml conical tube, the tube was filled with cold PBS up to 15 ml, and the content in the tube was centrifuged at 1200 rpm for 5 minutes and washed with PBS. This process was repeated three times to remove Matrigel. The cells were fixed with 4% (w/v) paraformaldehyde (PFA), incubated for 30 minutes, and then washed three times with PBS. Triton X-100 solution (in PBS; concentration: 1% (v/v)) was added to the cell sample and incubated for 1 hour, and then Triton X-100 solution (in PBS; concentration: 2% (v/v)) was added thereto, followed by washing twice. Then, blocking buffer (0.279 g of BSA, 450 µl of goat serum, 180 µl of Triton X-100, 450 µl of PBS 20X, 7,920 µl of DW) was added thereto, followed by incubation for 30 minutes. The resulting cells were treated with BubR1 antibody (BD Biosciences) and incubated for 16 hours. Then, the cells were washed three times with 0.2% (v/v) PBS-T (Triton X-100 solution in PBS) for 20 minutes each time. Then, the cells were incubated with HRP-tagged secondary antibody (Thermo Fisher Scientific) for 16 hours. Then, the cells were washed three times with 0.2%(v/v) PBS-T for 20 minutes each time, treated with FITC-conjugated tubulin antibody (Abcam) at 1:1000, and incubated for 2 days. The cells were washed three times with 0.2% (v/v) PBS-T for 20 minutes each time, and then incubated with DAPI for one day. After the supernatant was removed, the cells were transferred into a 8-well chamber and fluorescence images thereof were observed using a confocal microscope.

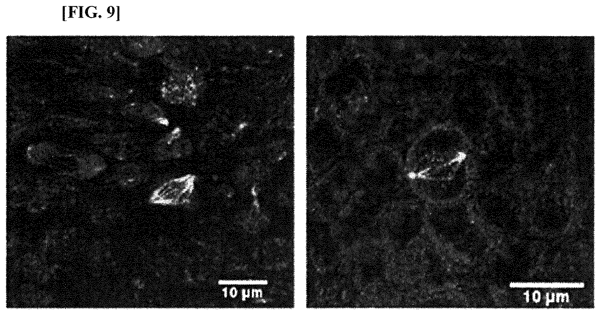

The observed fluorescence images are shown in FIG. 9. In FIG. 9, the left image shows the result obtained for pancreatic organoids derived from wild-type mice, and the right image shows the result obtained for pancreatic organoids derived from BubR1^{K243R/+} mice.

### Example 7. Test for Drug Responsiveness of BubR1^{K243R/+} Mouse Pancreatic Organoids

With reference to Examples 2 and 3, BubR1^{K243R/+} mouse pancreatic organoids were cultured, and then the drug responsiveness thereof was tested through the following steps:
Pancreatic tissue isolated from BubR1^{K243R/+} mice was dissociated, and cell pellets containing ductal cells were collected from the dissociated pancreatic tissue. The collected cell pellets were grown with a biomatrix in a cell incubator at 37°C under 5% CO₂ (see Examples 2 and 3). The obtained organoids were mechanically dissociated in consideration of the diameter of the end of the pipette, the volume of the culture medium, and the number of pressure applications. The mechanical stress is preferably 3 to 30,000 Pa, more preferably 3 to 3,000 Pa, even more preferably 50 to 3,000 Pa, most preferably 50 to 500 Pa. For example, when a pipette having an end diameter of 9 mm is used, the organoids are preferably pipetted 15 times with 1 ml of medium. Thereafter, the organoids were resuspended in Matrigel, seeded, and grown in an organoid culture medium to reach an organoid size of 100 to 200 µm. Thereafter, large organoids having a size of 200 µm or more were filtered out, and the remaining organoids were treated with a drug diluted in an organoid culture medium. The morphology of the organoids was observed with an inverted microscope and the viability thereof was measured through various assays.

The organoid culture medium had the following composition: A mixture of DMEM/F-12 (Dulbecco's Modified Eagle Medium/Ham's F-12; Thermo Fisher Scientific) with 1% (vol/vol) penicillin/streptomycin, 10 mM HEPES, 1% GlutaMAX, 1:50 B27 supplement (Gibco), 1 mM N-acetylcysteine, 5% (vol/vol) Rspo 1-conditioned medium (Hans Clevers lab), 10 mM nicotinamide, 10 nM recombinant human [Leu15]-gastrin I (Sigma Aldrich), 50 ng/ml of recombinant mouse EGF (Peptron), 100 ng/ml of recombinant human FGF10 (Peptron), and 25 ng/ml of recombinant human Noggin (Peptron) or 5% (vol/vol) Noggin-conditioned medium (Hans Clevers lab).

The organoids were treated with 5 µM of each of the panHDAC inhibitor AR-42 (N-hydroxy-4-[[(2S)-3-methyl-2-phenylbutanoyl]amino]benzamide; CAS No. 935881-37-1) and the HDAC6 inhibitor ACY-241 (Citarinostat; CAS No. 1316215-12-9) as drugs, and observed with an inverted microscope (Zeiss) on day3, and photographs obtained by the observation are shown in FIG. 10.

### Example 8. Development of Standardized Drug Treatment Method

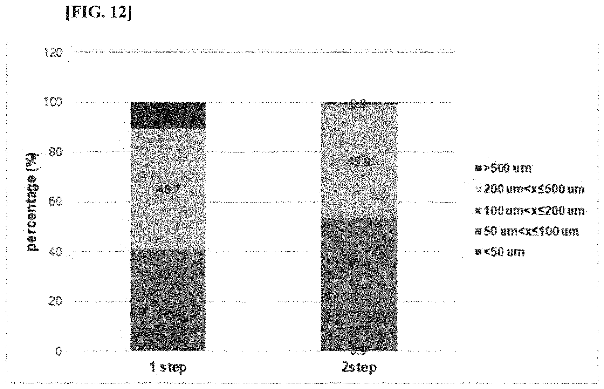

Pancreatic tissue isolated from the genetically mutated mice described in Examples 1 to 7 above was dissociated. Cell pellets containing ductal cells were collected from the dissociated pancreatic tissue. The collected cell pellets were grown with a biomatrix in a cell incubator at 37°C under 5% CO₂. Thereafter, the produced organoids were grown in an organoid culture medium until 75% or more thereof grew to a diameter of 200 to 500 µm. The grown organoids were mechanically dissociated with a mechanical stress of 50 to 500 Pa, and were grown with a biomatrix on a chip or plate in a cell incubator for one day at 37°C under 5% CO₂ so that 90% or more thereof had a diameter of 100 to 200 µm. The organoids were treated with a drug diluted in an organoid culture medium. After these procedures, the morphology of the organoids was observed with an inverted microscope and the viability thereof was measured by an MTT assay. As a result of the measurement, it could be confirmed that, when the above-described drug treatment method was used, it was possible to track the drug responsiveness of each organoid, which was a limitation of a conventional drug screening method using organoids, and drug treatment in a uniform size state was possible. FIG. 12 shows the results of comparing the size uniformity of organoids between the case in which organoids were subjected to a two-step process further including a step of physiologically dissociating the organoids by application of mechanical stress in a step of collecting and culturing cell pellets and the case in which organoids were not subjected to the two-step process. As the maximum value of the average diameter of the collected organoids increases, the difference in size (average diameter) between the organoids included increases, so that the difference in responsiveness to the same drug between the organoids increases. Thus, in order to obtain a uniform drug responsiveness result, it is important to determine the upper limit of the size (average diameter) of the organoids. It was confirmed that the present disclosure has a remarkable effect of increasing drug responsiveness by applying mechanical stress to the organoids and post-treating the organoids in two steps to uniformize the size of the obtained organoids. This process is expected to be available to obtain a uniform yield when culturing organoids derived from the pancreas as well as organoids derived from other tissues.

### Example 9. Observation of Responsiveness of Genetically Mutated Kras^{G12D} Mouse Organoids after Drug Treatment

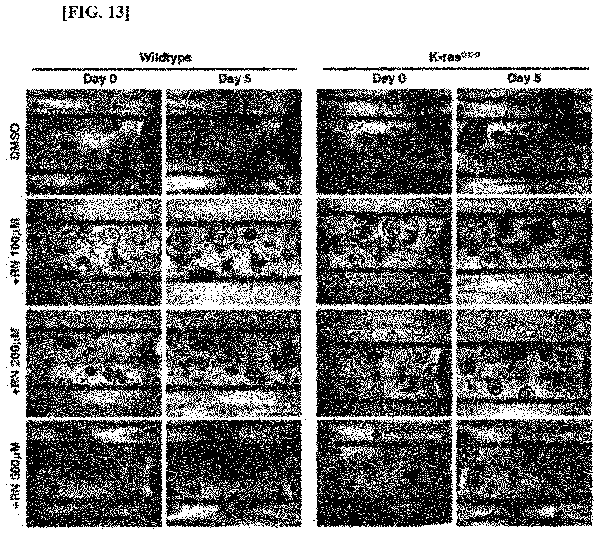

With reference to Example 8, pancreatic organoids with a uniform size were prepared from K-ras ^{G12D} mutant mice and wild-type mice, and the organoids were treated with 100 µM, 200 µM or 500 µM of Resveratrone drug. On day 5 after drug treatment, the organoids were observed with an inverted microscope (Zeiss), and images of observation are shown in FIG. 13. Resveratrone is a novel substance derived from resveratrol. It was confirmed that, when the organoids were grown on a common 12-well plate and treated with the drug as described in Example 5, the size of the organoids varied from 20 µm to 1,000 µm in diameter, and thus it was impossible to track each organoid, whereas, when the experiment was performed with reference to Example 8, there were advantages in that the organoids are seeded with a uniform size, and in that it is possible to track the shape and pattern of each organoid.

### Example 10. Optimization of Method of Measuring Viability of Genetically Mutated Kras^{G12D} Mouse Organoids after Drug Treatment

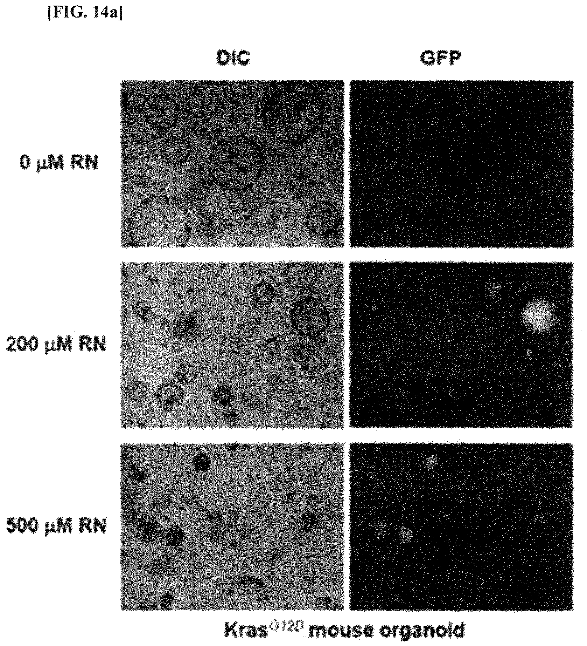

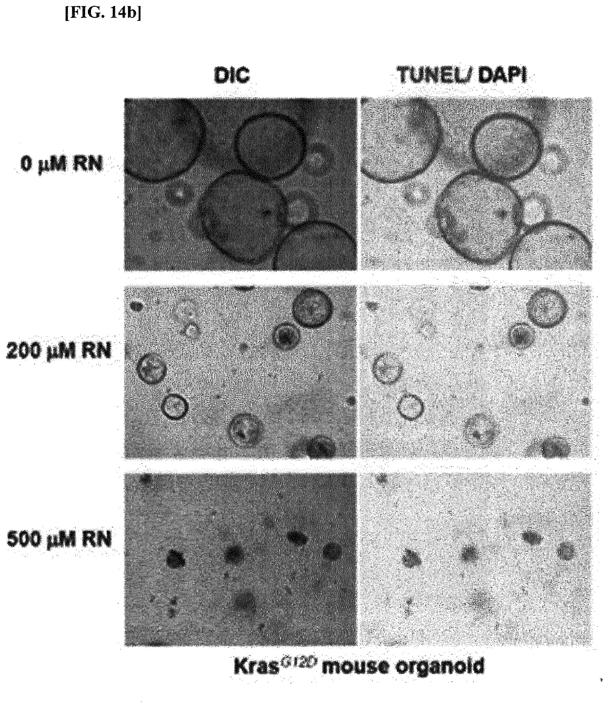

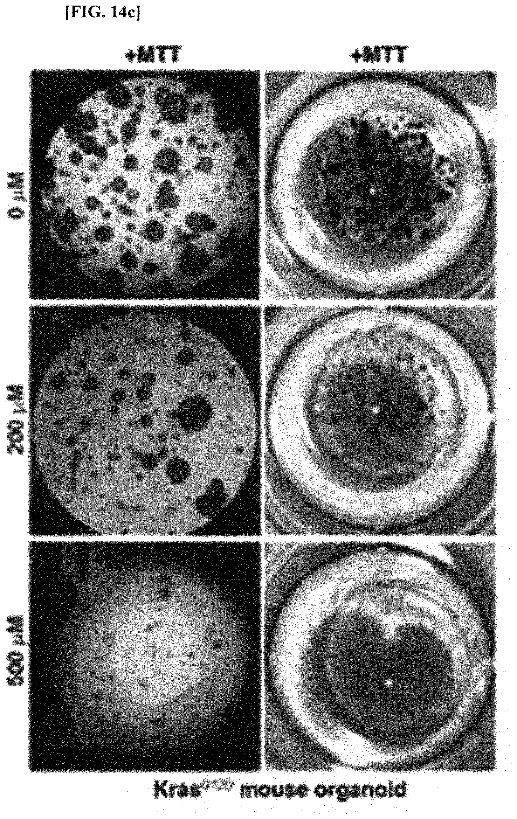

The TUNEL method is a method in which dying cells become fluorescent. As described in Example 9, with reference to Example 8, the pancreatic organoids from Kras^{G12D} mutant mice were treated with Resveratrone drug. The drug exhibits green fluorescence when cells are dying. The organoids were treated with 0 µM, 200 µM or 500 µM of the drug, followed by analysis of GFP (see FIG. 14a). As a result of the experiment, it could be confirmed that, when the pancreatic organoids from Kras^{G12D} mutant mice were treated with Resveratrone drug at a suitable concentration of 200 µM, they exhibited fluorescence, and when the organoids were treated with Resveratrone drug at a high concentration of 500 µM, cell death occurred and GFP decreased. At this time, it was found that the drug had no effect on normal pancreatic organoids. FIG. 14b shows the results of measuring the viability of the organoids by treatment with a TdT enzyme-containing staining solution and a DAPI reagent using a TUNEL assay on day 5 after treatment with the drug. The MTT method is a method of measuring metabolic activity, and is a method in which living cells become colored. As a result of the experiment, it could be seen that the reagent was not properly infiltrated into the organoids and thus the cells were not stained, indicating that it was impossible to confirm the viability of the cells by the above method. As described in Example 9, with reference to Example 8, the pancreatic organoids from Kras^{G12D} mutant mice were treated with Resveratrone drug. FIG. 14c shows the results of measuring the viability of the organoids by treatment with a staining solution using an MTT method on day 5 after the organoids were treated with 0 µM, 200 µM or 500 µM of Resveratrone drug.

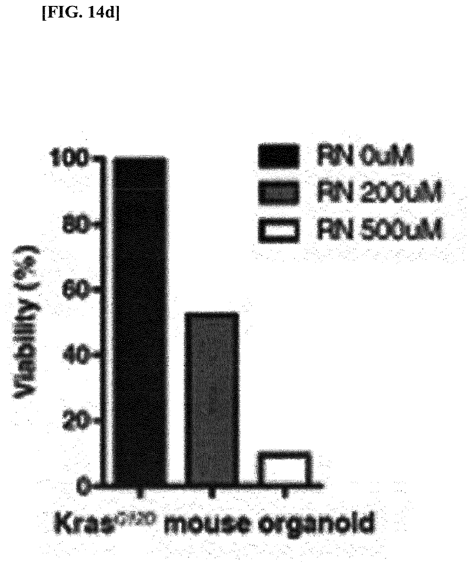

The results of measuring the viability of the organoids by the TUNEL method and the MTT method were compared. When the viability of the organoids was measured by the TUNEL method, there was no difference between those treated with the drug and those not treated with the drug (see FIGS. 14a and 14b). It can be confirmed that it was difficult to measure the viability because the reagent did not penetrate. On the other hand, when the viability of the drug-treated organoids was measured by applying the MTT method to the organoids, it could be confirmed that the number of viable cells increased, the color was darker, suggesting that the cells could be quantified (see FIG. 14d).

### Example 11. Verification of Drug Responsiveness of Pancreatic Organoids by Analysis of Expression Level of Specific Marker Using Western Blot Technique

The mouse-derived organoids described in Examples 1 to 7 or patient-derived organoids were primarily treated with a drug, and after 72 hours, secondarily treated with the drug. As the drug, Resveratrone used in Examples 9 and 10 was used. On day 2 after the secondary drug treatment (day 5 after the primary drug treatment), the cell culture medium was removed, and then the cells were washed with 1 ml of PBS. After removal of PBS, the cells were mechanically dissociated with 500 µl of a recovery solution. The cell solution was agitated at 50 to 70 rpm at 4°C for 1 hour, transferred into a microtube, and then centrifuged at 1200 rpm for 5 minutes, followed by removal of the supernatant. The remaining pellet was lysed with NETN buffer, and then sonicated using a sonicator for 1 minute with an output of 2 to 3 and centrifuged at 12,000 rpm for 20 to 30 minutes, and the supernatant was collected. The supernatant was quantified to 100 to 120 µg using Bradford assay, and then loaded onto a 10% polyacrylamide gel, followed by SDS-PAGE (Sodium Dodecyl Sulfate-Polyacrylamide gel electrophoresis) gel running. Then, the gel was transferred to a nitrocellulose membrane, and the transferred membrane was blocked with 5% skim milk while stirring at 50 rpm for 1 hour at RT. Using a Western blot technique, the membrane was treated with anti-p53 antibody at 4°C for 18 hours, and then treated with secondary antibody at RT for 3 hours, and then visualized using a film.

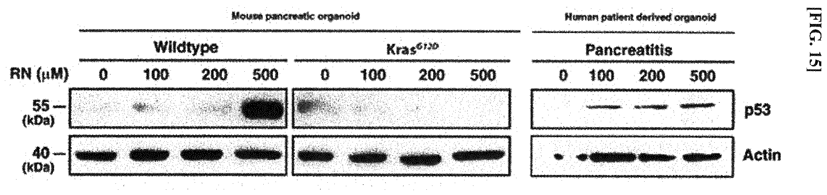

It is known that the tumor suppressor gene p53 inhibits abnormal proliferation of cells by regulating the cell cycle and induces apoptosis of cells such as cancer cells. Usually, p53 is inactivated in cancer cells, and referring to FIG. 15, it could be confirmed that, when the organoids were further treated with Resveratrone, responsiveness to the drug was specifically different between the mouse-derived normal organoids and the mutant organoids (cancer-like). It was confirmed that, when the normal organoids were treated with the drug, the expression level of p53 therein increased, whereas the expression level of p53 in the mutant organoids treated with the drug did not increase, and that this tendency was also found in the patient-derived organoids (see FIG. 15). Thereby, it is expected that p53 can be used as a marker to examine the responsiveness of normal cells and cancer cells to the drug by use of the corresponding technique.

### Industrial Applicability

Conventional organoid drug responsiveness test methods have a technical limitation in that uniform drug treatment is not achieved, resulting in poor accuracy. The present disclosure overcomes this limitation, and relates to more effective three-dimensional pancreatic organoids derived from cancer gene mutant mice and to a standardized method for evaluating drug effect. In order to provide personalized medicine to actual patients, it is required to develop a technology capable of efficiently verifying more uniform drug responsiveness. This experimental technique using the organoids according to the present disclosure provides a method of accurately verifying the effect of a candidate drug for cancer treatment by efficiently increasing the responsiveness of the organoids to the candidate drug. Therefore, applied research using these three-dimensional organoids will be a foothold that provides personalized medicine to actual patients.

### Sequence List Free Text

SEQ ID NO 1: CTCATCATTTGTTGCCTCACTTC
SEQ ID NO 2: TGTTGGATACAAGGCATGTAC
SEQ ID NO 3: GCACTCCTTACAAGGGACGA
SEQ ID NO 4: CTTCAATTTCCTTGGCTTCG
SEQ ID NO 5: ATTTGTCACGTCCTGCACGACG
SEQ ID NO 6: CGGCATGGTGCAAGTTGAAT
SEQ ID NO 7: CGGTGCTAACCAGCGTTTTC
SEQ ID NO 8: CTAGGCCACAGAATTGAAAGATCT
SEQ ID NO 9: GTAGGTGGAAATTCTAGCATCATCC
SEQ ID NO 10: GAGGTAAAGGCAGGGGAATC
SEQ ID NO 11: GAGAAAGCGGGGGTCATTAT

## Claims

1. A method for producing pancreatic organoids capable of continuous culture, the method comprising the steps of:
(a) isolating pancreatic tissue from a mouse, wherein the mouse is genetically modified by knockout of the telomerase RNA component (TERC) and by the deletion of exon 11 of the BRCA2 gene;
(b) mixing cells isolated from the tissue with Matrigel, and culturing the mixed cells to form pre-organoids;
(c) dissociating the pre-organoids with a mechanical stress of 50 to 500 Pa, and
(d) re-culturing the dissociated organoids by repeated passaging.

2. The method of claim 1, wherein the organoids have a diameter of 100 to 200 µm.

## Patentansprüche

1. Verfahren zur Herstellung von Organoiden der Bauchspeicheldrüse, die für eine kontinuierliche Kultur geeignet sind, wobei das Verfahren die Schritte umfasst:
(a) Isolieren von Pankreasgewebe aus einer Maus, wobei die Maus durch Knockout der Telomerase-RNA-Komponente (TERC) und durch die Deletion von Exon 11 des BRCA2-Gens genetisch verändert ist,
(b) Mischen von aus dem Gewebe isolierten Zellen mit Matrigel und Kultivieren der gemischten Zellen zur Bildung von Prä-Organoiden,
(c) Dissoziieren der Prä-Organoide mit einem mechanischen Stress von 50 bis 500 Pa und
(d) Rekultivierung der dissoziierten Organoide durch wiederholtes Passagieren.

2. Verfahren nach Anspruch 1, wobei die Organoide einen Durchmesser von 100 bis 200 µm haben.

## Revendications

1. Procédé de production d'organoïdes pancréatiques capables d'être cultivés en continu, le procédé comprenant les étapes de :
(a) isolement de tissu pancréatique d'une souris, dans lequel la souris est génétiquement modifiée par l'élimination du composant ARN de la télomérase (TERC) et par la suppression de l'exon 11 du gène BRCA2 ;
(b) mélange de cellules isolées du tissu avec du Matrigel et culture des cellules mélangées pour former des pré-organoïdes ;
(c) dissociation des pré-organoïdes avec une contrainte mécanique de 50 à 500 Pa, et
(d) re-culture des organoïdes dissociés par passages répétés.

2. Procédé de la revendication 1, dans lequel les organoïdes comportent un diamètre de 100 à 200 µm.
